# EUROPEAN PATENT APPLICATION

(11) **EP 4 850 688 A1**
(43) Date of publication of application: **09.12.2026**
(21) Application number: 25748050.9
(22) Date of filing: 05.02.2025
(51) Int. Cl.: A61K 39/29, A61P 31/20, C12N 15/62, C12N 15/85, C07K 16/08

(54) **PREPARATION OF NEW MRNA VACCINE, AND USE THEREOF IN PHARMACEUTICAL COMBINATION FOR TREATING HEPATITIS B VIRUS DISEASE**

(30) Priority: 02.02.2024 CN 202410149422
(71) Applicant: Nanjing Yuhang Biopharmaceutical Co., Ltd., Nanjing City, Jiangsu Province 211112 (CN)
(72) Inventor: YANG, Meijia, Nanjing, Jiangsu 211112 (CN); LI, Wenguo, Nanjing, Jiangsu 211112 (CN)
(74) Representative: Lavoix
(86) International application number: PCT/CN2025/075833
(87) International publication number: WO 2025/162490

(57) **Abstract**

Provided in the present invention are a preparation of a new mRNA vaccine, and the use thereof in a pharmaceutical combination for treating the hepatitis B virus disease. Specifically, provided in the present invention is a therapeutic mRNA vaccine against chronic hepatitis B infection, which vaccine contains an mRNA encoding a large protein (L protein) region of a hepatitis B virus surface antigen, and also contains two mRNAs respectively encoding a middle protein (M protein) region of the hepatitis B virus surface antigen and encoding a small protein (S protein) region thereof, or contains an mRNA encoding the small protein (S protein) region. The therapeutic mRNA vaccine of the present invention can break the immune tolerance state in chronic hepatitis B infection, and has the potential to functionally cure chronic hepatitis B infection clinically.

## Description

### Technical field

The present invention relates to the technical field of mRNA vaccines. Specifically, the present invention relates to the preparation of a novel mRNA vaccine and uses thereof in a pharmaceutical combination for treating hepatitis B virus diseases.

### Background

According to statistics, there are approximately 300 million patients with chronic hepatitis B (CHB) infection worldwide, which is a global disease seriously endangering human health. At present, clinical drugs for treating CHB include interferons and nucleotide analogues, which exert therapeutic effects by activating innate immunity and inhibiting RNA in the HBV replication cycle respectively. Monotherapy with either drug is only effective in a small subset of patients, and a combined therapy can improve the functional cure rate, yet therapeutic efficacy varies greatly among hepatitis B patients with different clinical characteristics. Massive continuous stimulation of the immune system by hepatitis B surface antigen (HBsAg) from HBV in CHB patients induces immune tolerance, the body stops producing HBsAb, the antibody targeting the surface antigen, and T cell immune responses are also suppressed, thereby rendering HBV extremely difficult to be eliminated. Reactivating HBsAb production, breaking immune tolerance, and subsequently restoring antiviral immune responses serve as the key and prerequisite for CHB treatment.

Breaking immune tolerance, which is marked by sustained high-level production of HBsAb, requires the reactivation of B cells and maintenance of antibody production. Controlling viral infection and replication, suppressing elevated HBsAg levels, and preventing persistent exhaustion of HBsAb and B cells constitute the keys to resolve the issue. The N-terminal Pre-S1 region of the HBV large surface protein (L protein) mediates virus binding to the hepatocyte surface receptor NTCP and subsequent viral entry. In patients with chronic hepatitis B, the antibody level against Pre-S1 is positively correlated with the cure rate and negatively correlated with cccDNA load. Therefore, specific activation of antibodies targeting Pre-S1 serves as a prerequisite for breaking immune tolerance. Pre-S1 or Pre-S polypeptides fail to effectively inhibit persistent viral infection and replication. The L protein possesses the native Pre-S antigen structure; however, it cannot be stably expressed on cell membranes or secreted alone, while only resides in the endoplasmic reticulum or Golgi apparatus and possesses cytotoxicity. HBV virus-like particles (VLPs) contain S protein, M protein and membrane components, which can effectively support the L protein. LMS VLPs expressed and secreted by mammalian cells, when combined with adjuvants, can trigger the production of antibodies against Pre-S1 and S protein, yet the HBsAg clearance rate remains low. In LMS VLPs, there is the highest abundance of S protein, followed by M protein, with the lowest level of L protein. Therefore, insufficient activation by L protein antigen may lead to the above outcome. In addition, the preparation of LMS VLPs involves procedures such as gene transduction, monoclonal screening, fermentation and protein purification, leading to long production cycles as well as complicated manufacturing costs and quality control.

Advantages of mRNA vaccines lie in the short design and preparation cycles, low costs, and low complexity in production and purification processes. More importantly, mRNA modification technologies and LNP delivery systems have overcome obstacles related to immunotoxicity and expression stability, supporting broad application prospects. Unlike recombinant protein or inactivated virus vaccines, mRNA vaccines require no adjuvants and do not introduce corresponding side effects; they can intrinsically activate a certain level of innate immune responses to boost immunization efficacy. Unlike virus vector-based or DNA-based antigen expression approaches, mRNA has no risk of integration into host cell DNA, which undergoes gradual degradation after intracellular translation and ceases expression once sufficient antibodies and immune memory are generated following repeated immunization. Current studies have generated mRNA encoding the L protein, as well as mRNA fusing sequences encoding Pre-S1 with other HBV antigens, soluble sPD-1 and soluble sCD40L, for treating HBV mice. Results indicate that L mRNA exerts effects only when combined with antiviral drugs, whereas Pre-S1-fused mRNA failed to yield critical antibody data. Therapeutic results using mRNA encoding full-length HBsAg demonstrated elevated HBsAb levels and reduced the surface antigen concentrations, yet this study was not conducted in HBV transgenic Tg mice with complete immune tolerance.

Therefore, there is a need in the field to develop a therapeutic hepatitis B vaccine capable of generating sufficient antibodies and breaking immune tolerance.

### Summary of the invention

The purpose of the present invention is to provide a therapeutic hepatitis B vaccine capable of generating sufficient antibodies and breaking immune tolerance.

In the first aspect of the present invention, a vaccine composition is provided, comprising:
a first polynucleotide N_{L} encoding hepatitis B virus surface antigen L protein; and
a second polynucleotide N_{S} encoding hepatitis B virus surface antigen S protein.

In another preferred embodiment, the vaccine composition further comprises a third polynucleotide N_{M} encoding hepatitis B virus surface antigen M protein.

In another preferred embodiment, the L protein comprises an N-terminal Pre-S region and a C-terminal S protein region.

In another preferred embodiment, the M protein comprises an N-terminal Pre-S2 region and a C-terminal S protein region.

In another preferred embodiment, the amino acid sequence of the L protein is shown in SEQ ID NO: 1.

In another preferred embodiment, the amino acid sequence of the M protein is shown in SEQ ID NO: 2.

In another preferred embodiment, the amino acid sequence of the S protein is shown in SEQ ID NO: 3.

In another preferred embodiment, in the vaccine composition, the polynucleotides encoding immunogens consist only of N_{L} and N_{S}, or only of N_{L}, N_{M} and N_{S}.

In another preferred embodiment, in the vaccine composition, the molar ratio of N_{L} to N_{S} is 1:10 to 10:1, preferably 1:5 to 5:1, more preferably 1:2 to 2:1, such as 1:1.

In another preferred embodiment, in the vaccine composition, the molar ratio of N_{L} to N_{M} is 1:10 to 10:1, preferably 1:5 to 5:1, more preferably 1:2 to 2:1, such as 1:1.

In another preferred embodiment, each of the polynucleotides N_{L}, N_{M} and N_{S} is independently DNA or mRNA.

In another preferred embodiment, the nucleic acid sequences encoding the L protein, M protein and/or S protein are derived from nucleic acid sequences corresponding to hepatitis B virus surface antigens of the same or different genotypes.

In another preferred embodiment, the nucleic acid sequences of N_{L}, N_{M} and/or N_{S} are codon-optimized.

In another preferred embodiment, the encoding nucleic acid sequences of N_{L}, N_{M} and/or N_{S} are optimized or modified, and such optimization or modification will not alter the antigen epitope in the Pre-S region for receptor binding.

In another preferred embodiment, the transcription start site of M protein and S protein in the L protein coding sequence is mutated, so that only the L protein can be translated.

In another preferred embodiment, the transcription start site of S protein in the M protein coding sequence is mutated, so that only the M protein can be translated.

In another preferred embodiment, the polynucleotides N_{L}, N_{M} and N_{S} are mRNA.

In another preferred embodiment, the mRNA sequence encoding the L protein in N_{L} is shown in SEQ ID NO: 4.

In another preferred embodiment, the mRNA sequence encoding the M protein in N_{M} is shown in SEQ ID NO: 5.

In another preferred embodiment, the mRNA sequence encoding the S protein in N_{S} is shown in SEQ ID NO: 6.

In another preferred embodiment, N_{L} and N_{S} are fused into the same mRNA.

In another preferred embodiment, N_{L}, N_{M} and N_{S} are fused into the same mRNA.

In another preferred embodiment, the mRNA has the structure of Formula I:

Z1-Z2-Z3-Z4-Z5-Z6 (I)

wherein,
Z1 is absent or a 5' capping element;
Z2 is a 5'-UTR element;
Z3 is absent or a signal peptide coding sequence;
Z4 is the coding sequence of hepatitis B virus surface antigen protein, wherein the hepatitis B virus surface antigen protein is selected from the group consisting of L protein, M protein, S protein, or a combination thereof;
Z5 is a 3'-UTR element;
Z6 is a polyA tail element.

In another preferred embodiment, the 5' capping element is cap0 or cap1.

In another preferred embodiment, Z4 comprises a polynucleotide sequence selected from the group consisting of the sequences set forth in SEQ ID NO: 4, 5 and 6, or a combination thereof.

In another preferred embodiment, the Z4 sequence is shown in any one of SEQ ID NO: 4, 5 and 6.

In another preferred embodiment, the Z4 has a structure represented by the following formula (II):

N1-L1-N2-L2-N3 (II)

wherein,
N1, N2 and N3 are each independently absent, or an N_{L} sequence, or an N_{M} sequence, or an N_{S} sequence;
L1 and L2 are each independently a coding sequence of a self-cleaving linker fragment;
and at least one of N1, N2 and N3 is an N_{L} sequence, and at least one is an N_{S} sequence.

In another preferred embodiment, the self-cleaving linker fragment is selected from the group consisting of IRES, P2A, and a combination thereof.

In another preferred embodiment, the mRNA is a modified mRNA; preferably, the modification comprises pseudouridine modification.

In another preferred embodiment, the polynucleotide is loaded in a vector.

In another preferred embodiment, N_{L} and N_{S} are loaded in the same vector or different vectors.

In another preferred embodiment, N_{L}, N_{M} and N_{S} are loaded in the same vector or different vectors.

In another preferred embodiment, the vector is selected from the group consisting of: a lipid nanoparticle (LNP), polymer particle, bacterial plasmid, bacteriophage, yeast plasmid, plant cell viruse, mammalian cell viruse, lentiviral vectors, adenoviral vectors, adeno-associated viral vectors, and a combination thereof.

In another preferred embodiment, the vector is a plasmid.

In another preferred embodiment, the vector is an adeno-associated viral vector.

In another preferred embodiment, the vector is a lipid nanoparticle (LNP).

In another preferred embodiment, the vaccine composition is a lipid nanoparticle, which comprises an outer liposome layer and the polynucleotide encapsulated therein, and preferably, the polynucleotide is mRNA.

In another preferred embodiment, mRNAs encoding different hepatitis B virus surface antigen proteins are encapsulated in the same LNP or different LNPs.

In another preferred embodiment, the components of the liposome layer are selected from the group consisting of: cationic lipid (such as SM-102), auxiliary lipid (such as DSPC), cholesterol, PEG-modified lipid (such as PEG2000-DMG), and a combination thereof.

In another preferred embodiment, the liposome layer comprises or consists of the following components: cationic lipid SM-102, auxiliary lipid DSPC, cholesterol and PEG2000-DMG lipid.

In another preferred embodiment, on a molar ratio basis, the liposome layer comprises or consists of the following components:
40-60% (preferably 50-55%) cationic lipid SM-102;
5-15% (preferably 10-12%) auxiliary lipid DSPC;
30-45% (preferably 35%-38.5%) cholesterol; and
0.5-2% (preferably 1-1.5%) PEG2000-DMG lipid.

In another preferred embodiment, the average particle size of the liposomal nanoparticles is 50-250 nm.

In another preferred embodiment, the polydispersity index (PDI) of the liposomal nanoparticles is 0.01-0.3.

In another preferred embodiment, the vaccine composition further comprises an immunostimulatory factor, or a polynucleotide encoding the immunostimulatory factor.

In another preferred embodiment, the immunostimulatory factor is selected from the group consisting of CD40L protein, mitochondrial antiviral signaling protein (MAVS protein), CD79a, CD79b, CD19, CD21 and CD81 protein.

In another preferred embodiment, the immunostimulatory factor serves as an auxiliary signal for B cell activation, a second signal for B cell activation or a co-stimulatory signal for B cell activation, enhancing the intensity of immune response and antiviral effect of mRNA vaccine.

In another preferred embodiment, the immunostimulatory factor is a protein or polypeptide.

In another preferred embodiment, the polynucleotide encoding the immunostimulatory factor is mRNA or DNA.

In another preferred embodiment, the polynucleotide encoding the immunostimulatory factor is loaded in a vector, preferably a plasmid or a virus.

In another preferred embodiment, after the vaccine composition transfects a cell, the hepatitis B virus surface antigen L protein, S protein and optional M protein are stably expressed on the cell surface.

In another preferred embodiment, the vaccine composition further comprises a pharmaceutically acceptable diluent, excipient, adjuvant, and a combination thereof.

In another preferred embodiment, the vaccine composition comprises no adjuvant.

In another preferred embodiment, the vaccine composition is in a liquid dosage form, solid dosage form, or gel dosage form.

In another preferred embodiment, the vaccine composition is administered via a route selected from the group consisting of subcutaneous injection, intradermal injection, intramuscular injection, intravenous injection, intraperitoneal injection, microneedle injection, oral administration, oronasal spray and aerosol inhalation.

In the second aspect of the present invention, an engineered host cell is provided, wherein the engineered host cell expresses membrane-bound hepatitis B virus surface antigen L protein and S protein.

In another preferred embodiment, the host cell is obtained by transfection with the vaccine composition according to the first aspect of the present invention.

In another preferred embodiment, the host cell further expresses membrane-bound hepatitis B virus surface antigen M protein.

In another preferred embodiment, the host cell further expresses an immunostimulatory factor.

In another preferred embodiment, the immunostimulatory factor is selected from the group consisting of CD40L protein, mitochondrial antiviral signaling protein (MAVS), CD79a, CD79b, CD19, CD21 and CD81 protein.

In another preferred embodiment, the hepatitis B virus surface antigen expressed by the host cell activates B cell immunity.

In another preferred embodiment, the host cell is selected from the group consisting of: a muscle cell, macrophage, dendritic cell, fibroblast, endothelial cell, epithelial cell, adipocyte, neutrophil and lymphocyte.

In the third aspect of the present invention, a method for preparing the engineered host cell according to the second aspect of the present invention is provided, comprising a step of: transducing the vaccine composition according to the first aspect of the present invention into an immune cell, so as to obtain the engineered host cell.

In the fourth aspect of the present invention, use of the vaccine composition according to the first aspect of the present invention or the engineered host cell according to the second aspect of the present invention in the preparation of a medicament is provided, wherein the medicament is used in a subject for:
(a) preventing hepatitis B virus infection;
(b) inducing the production of antibodies specific for hepatitis B virus; and/or
(c) treating chronic hepatitis B infection.

In another preferred embodiment, the medicament is a vaccine.

In another preferred embodiment, the engineered host cell is an autologous or allogeneic cell relative to the subject.

In another preferred embodiment, the chronic hepatitis B is immune-tolerant chronic hepatitis B.

In the fifth aspect of the present invention, a medicament for treating chronic hepatitis B infection is provided, wherein the medicament comprises the vaccine composition according to the first aspect of the present invention or the host cell according to the second aspect of the present invention.

In another preferred embodiment, the medicament is a vaccine.

In another preferred embodiment, the vaccine induces the production of antibodies targeting native Pre-S antigen epitopes in chronic hepatitis B infection.

In another preferred embodiment, the chronic hepatitis B is immune-tolerant chronic hepatitis B.

In the sixth aspect of the present invention, a method for preventing and/or treating hepatitis B is provided, comprising a step of:
administering the vaccine composition according to the first aspect of the present invention or the engineered immune cell according to the second aspect of the present invention to a subject in need thereof.

In another preferred embodiment, the subject comprises a human or non-human mammal.

In another preferred embodiment, the non-human mammal comprises a rodent and non-human primate.

In the seventh aspect of the present invention, a method for generating anti-hepatitis B virus antibodies is provided, comprising a step of:
immunizing an animal with the vaccine composition according to the first aspect of the present invention or the host cell according to the second aspect of the present invention, thereby generating the anti-hepatitis B virus antibodies.

In another preferred embodiment, the animal comprises a human and non-human mammal (such as a rodent, e.g., mouse and rat).

In another preferred embodiment, the antibody is an antibody against the Pre-S antigen epitope.

It should be understood that within the scope of the present invention, the above various technical features of the present invention and the various technical features specifically described hereinafter (such as in the examples) can be combined with one another to form new or preferred technical solutions, which will not be enumerated one by one herein due to limited contents.

### Description of drawings

Following drawings are used to illustrate specific embodiments of the present invention, instead of limiting the scope of the present invention as defined by the claims.
Figure 1 shows the proportion of cells positive for the Pre-S2 antigen on the monoclonal cell surface detected by flow cytometry.
Figure 2 shows the expression levels of L protein and S protein in total protein, cell membrane components and cytoplasmic components of monoclonal cells detected by Western blot.
Figure 3 shows the distribution of membrane raft structures on the surface of monoclonal cells observed by transmission electron microscopy.
Figure 4 shows the integrity of L mRNA, M mRNA and S mRNA detected separately by capillary electrophoresis.
Figure 5 shows the particle sizes of L mRNA, M mRNA and S mRNA complexes encapsulated by LNPs detected separately by dynamic light scattering.
Figure 6 shows the surface potentials of L mRNA, M mRNA and S mRNA complexes encapsulated by LNPs detected by phase analysis light scattering, respectively.
Figure 7 shows the physicochemical properties, encapsulation efficiency and endotoxin test results of L mRNA, M mRNA and S mRNA complexes encapsulated by LNPs.
Figure 8 shows the proportions of cells positive for Pre-S2 and S antigens on the cell surface determined by flow cytometry after *in vitro* transfection of 293T cells with L mRNA, M mRNA and S mRNA.
Figure 9 shows the expression levels of target proteins in cells and culture supernatants detected by Western blot after *in vitro* transfection of 293T cells with L mRNA, M mRNA and S mRNA.
Figure 10 shows the protein characteristic analysis and VLP characterization of purified LMS VLPs.
Figure 11A shows the HBsAb levels in HBV Tg mice immunized with mRNA vaccine.
Figure 11B shows the trend of anti-S antigen antibody titers in HBV Tg mice immunized with mRNA vaccine.
Figure 11C shows the trend of anti-preS antigen antibody titers in HBV Tg mice immunized with mRNA vaccine.
Figure 12 shows individual HBsAg levels in HBV Tg mice immunized with mRNA vaccine.
Figure 13 shows the correlation analysis between the reduction rate of HBsAg and HBsAb levels in HBV Tg mice immunized with mRNA vaccine.
Figure 14 shows the levels of HBsAg, HBsAb and HBeAg in the pAAV HBV1.2 mouse model immunized with mRNA vaccine.
Figure 15 shows the binding of purified IgG from serum of mRNA vaccine-immunized HBV Tg mice to Pre-S peptides, recombinant HBsAg protein and LMS VLPs detected by indirect ELISA.
Figure 16, panel a shows 293 monoclonal cells with positive surface Pre-S2 antigen selected by flow sorting.

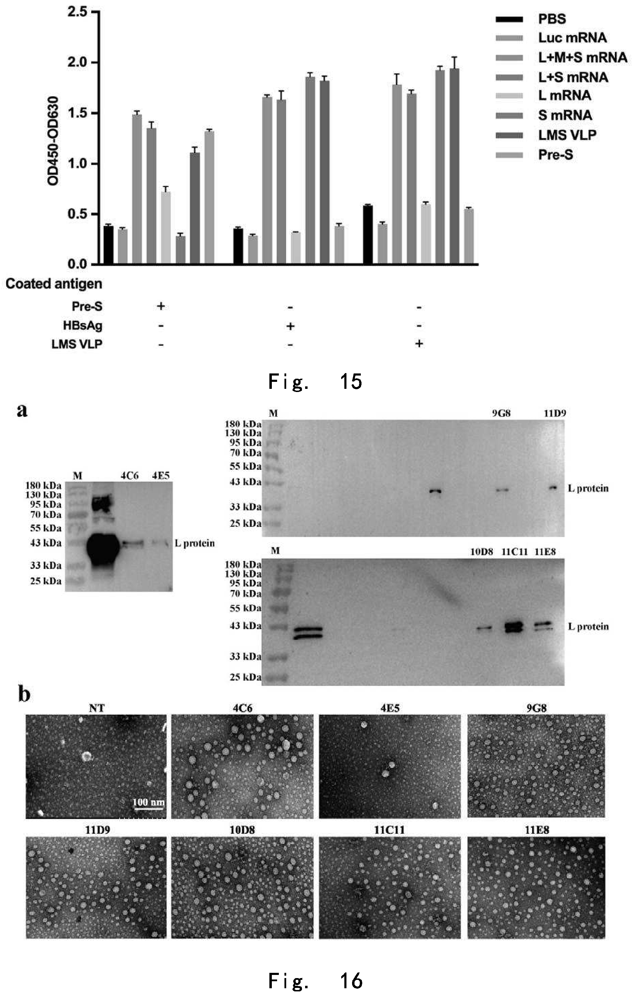

Figure 16, panel b shows VLPs produced by 293 monoclonal cells expressing L+S observed under a scanning electron microscope.

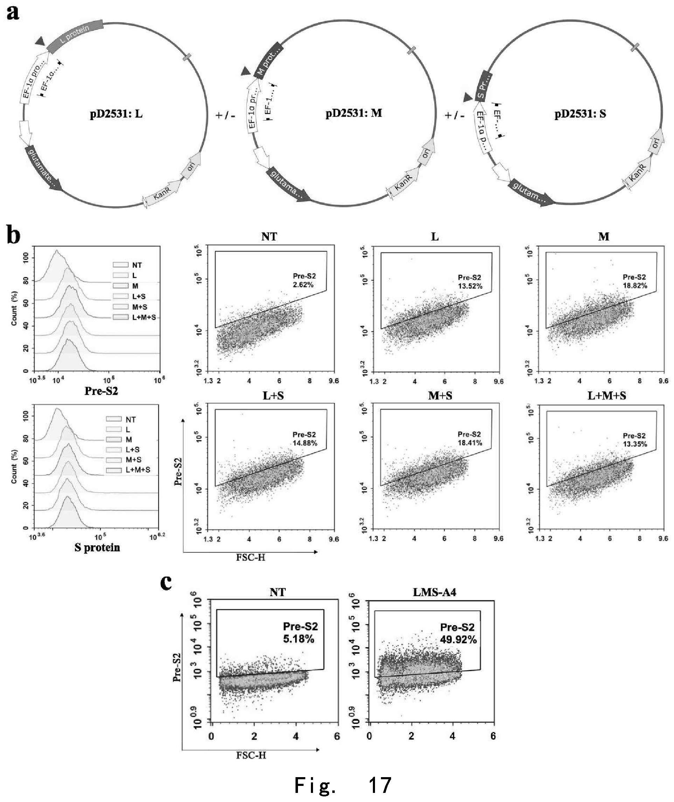

Figure 17, panel a is a schematic diagram of the plasmids used for transducing 293 cells.
Figures 17, panel b and panel c show flow cytometry detection of hepatitis B antigen expressed on the surface of 293 cells.

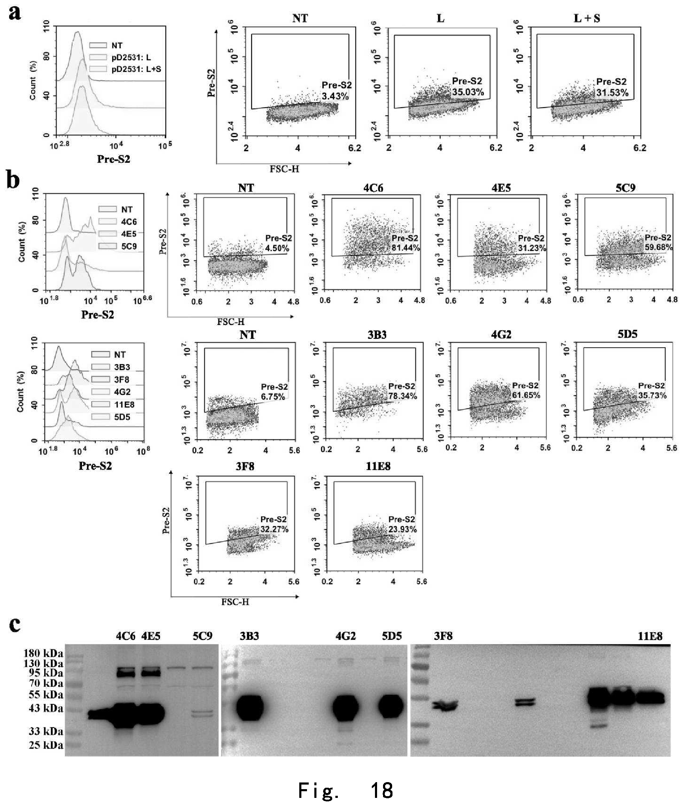

Figure 18, panel a shows surface L antigen expression in stable 293 cell lines transduced with L or L+S plasmids.
Figure 18, panel b shows flow cytometry detection of L antigen expression in stable 293 cell lines transduced with L+S plasmids.
Figure 18, panel c shows Western blot detection of L antigen expression in stable 293 cell lines transduced with L+S plasmids.

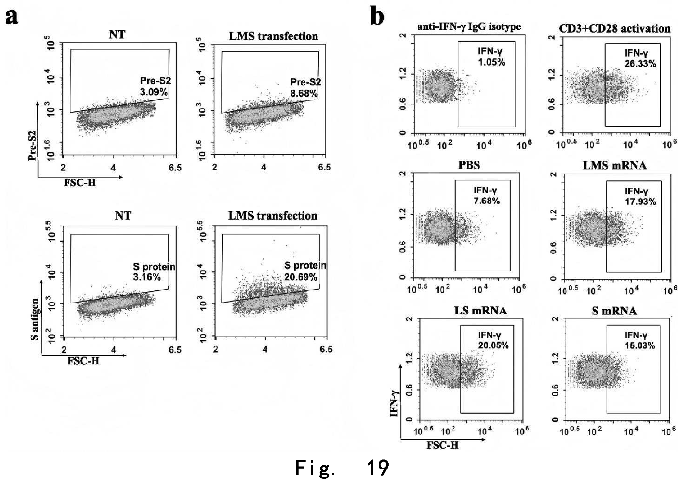

Figure 19, panel a shows the flow cytometry detection results of preS antigen and S antigen expression after co-transducing mouse embryonic fibroblast Balb/3T3 cells with three plasmids L, M and S.
Figure 19, panel b shows the proportion of IFN-γ-positive cells among CD3-positive cells detected by flow cytometry after co-incubation of T cells with Balb/3T3 cells expressing LMS antigens.

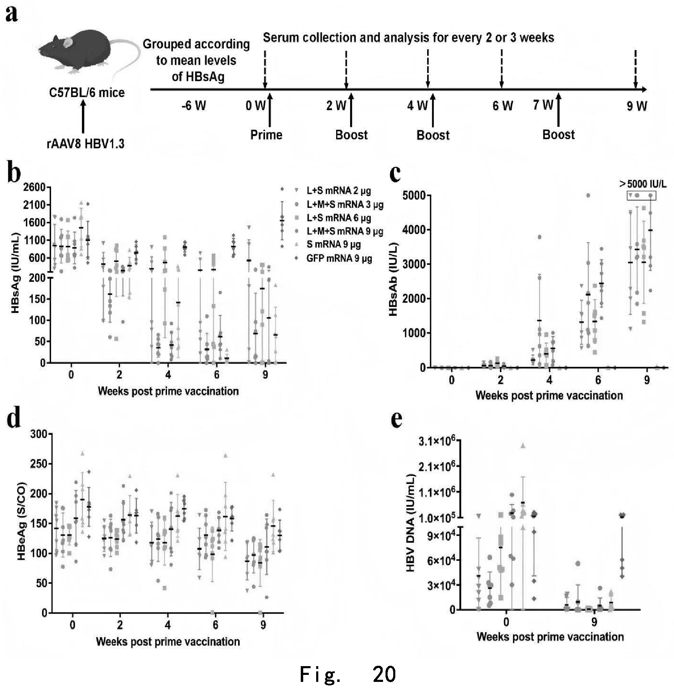

Figure 20, panel a shows the schematic diagram of the experimental protocol for immunizing rAAV8 HBV1.3 mice (1×10¹ vg/mouse) with different doses of LS, LMS mRNA, S mRNA or GFP mRNA.
Figure 20, panel b shows the changes in HBsAg antigen in rAAV8 HBV1.3 mice (1×10¹ vg/mouse) immunized with different mRNAs.
Figure 20, panel c shows the changes in anti-HBsAb antibody in rAAV8 HBV1.3 mice (1×10¹ vg/mouse) immunized with different mRNAs.
Figure 20, panel d shows the changes in HBeAg antigen in rAAV8 HBV1.3 mice (1×10¹ vg/mouse) immunized with different mRNAs.
Figure 20, panel e shows the changes in HBV DNA levels in rAAV8 HBV1.3 mice (1×10¹ vg/mouse) immunized with different mRNAs.

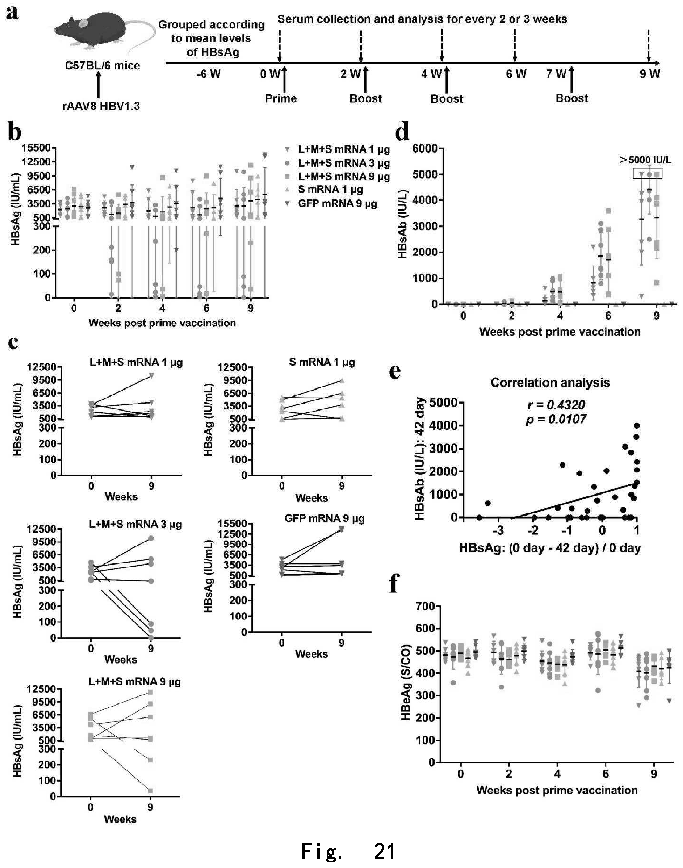

Figure 21, panel a is a schematic diagram of the experimental protocol for immunizing rAAV8 HBV1.3 (5×10¹ vg/mouse) mice with different doses of LMS mRNA, S mRNA or GFP mRNA.
Figure 21, panel b shows the changes in serum HBsAg antigen in the mice in Figure 20a after mRNA immunization.
Figure 21, panel c shows the comparison of serum HBsAg antigen at week 0 and week 9 in the mice in Figure 20a after mRNA immunization.
Figure 21, panel d shows the changes in serum anti-HBsAg antibodies in the mice in Figure 20a after mRNA immunization.
Figure 21, panel e shows the correlation analysis between the activation level of serum HBsAb and the reduction level of HBsAg at week 0 and week 6.
Figure 21, panel f shows the detection of serum HBeAg changes in mice from week 0 to week 9.

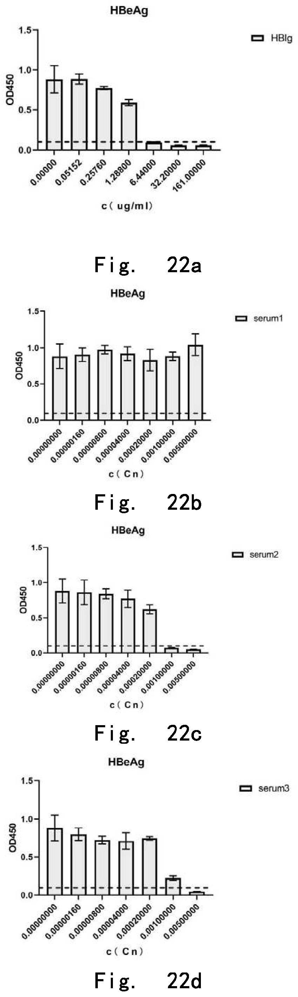

Figure 22a shows the inhibitory effect of positive control HBIg on HBeAg expression in the *in vitro* HBV-infected HepG2-hNTCP cell line model.
Figure 22b shows the inhibitory effect of negative control serum on HBeAg expression in the *in vitro* HBV-infected HepG2-hNTCP cell line model.
Figure 22c shows the inhibitory effect of LMS mRNA immunized serum on HBeAg expression in the *in vitro* HBV-infected HepG2-hNTCP cell line model.
Figure 22d shows the inhibitory effect of LS mRNA immunized serum on HBeAg expression in the *in vitro* HBV-infected HepG2-hNTCP cell line model.

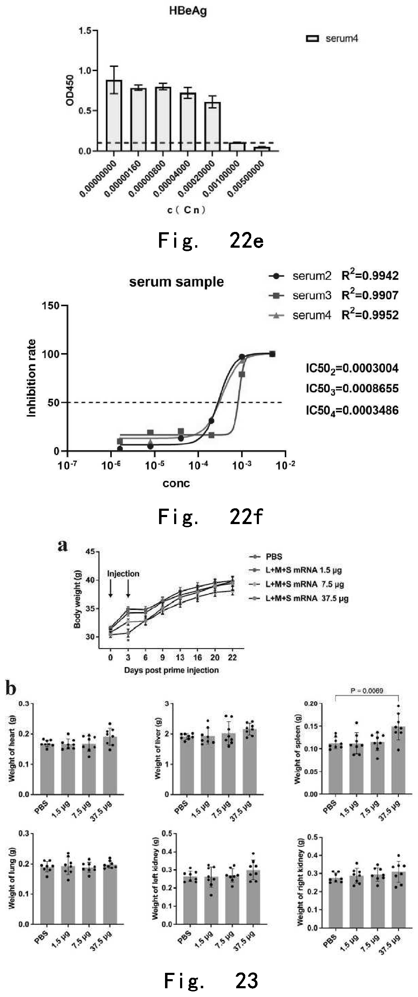

Figure 22e shows the inhibitory effect of S mRNA immunized serum on HBeAg expression in the *in vitro* HBV-infected HepG2-hNTCP cell line model.
Figure 22f shows the comparison and conversion against the specific IC50 concentration value of the HBIg positive control antibody, thereby calculating the effective neutralizing antibody concentration in the original serum sample.
Figure 23, panel a shows the changes in the body weight in mice immunized with different doses of LMS mRNA or PBS during the acute toxicity test of the LMS mRNA vaccine in mice.
Figure 23, panel b compares the weights of the heart, liver, spleen, lung and kidney of mice at the end of the acute toxicity test. Representative results are expressed as mean ± standard deviation (SD).

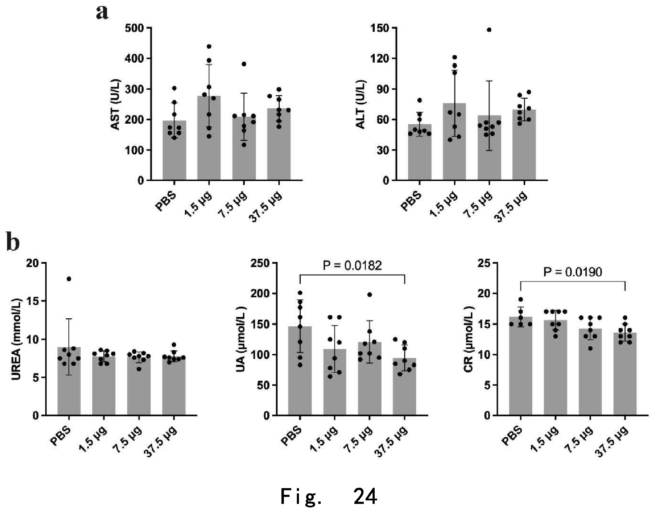

Figure 24, panel a shows the analysis of liver and kidney functions in the acute toxicity test of the LMS mRNA vaccine, including the levels of liver function indicators aspartate aminotransferase (AST) and alanine aminotransferase (ALT) at the end of the acute toxicity test.
Figure 24, panel b shows the renal function indicators at the end of the acute toxicity test, including the levels of urea, uric acid (UA) and creatinine (CR). Representative results are expressed as mean ± standard deviation (SD).

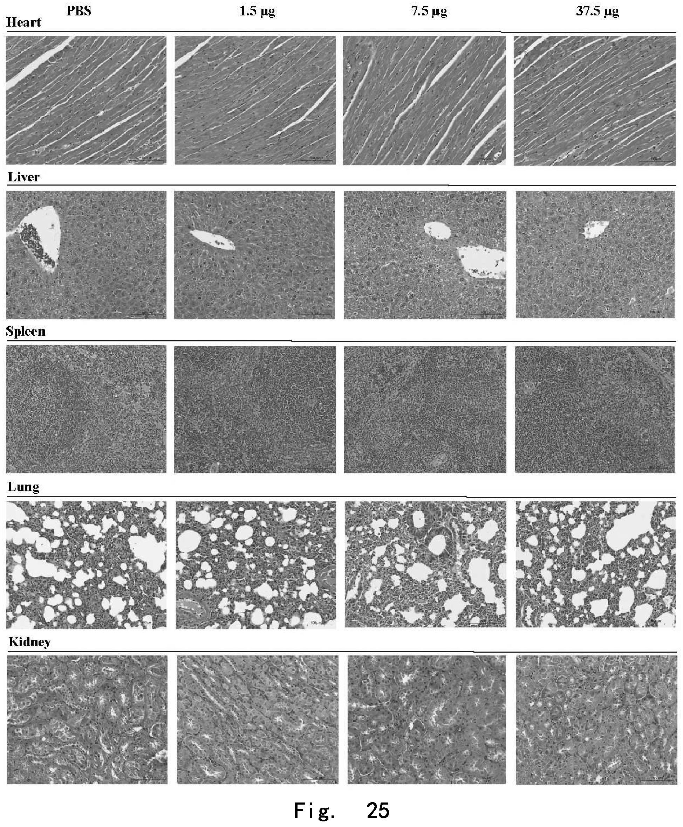

Figure 25 shows H&E staining and pathological analysis of major organs (heart, liver, spleen, lung and kidney) from mice immunized with different doses of LMS mRNA or PBS at the end of the acute toxicity test.

### Detailed Description

After extensive and in-depth research, the present inventors have developed for the first time a preparation method for a novel mRNA vaccine and uses thereof in a pharmaceutical combination for treating hepatitis B virus-related diseases. Specifically, the present invention provides a therapeutic mRNA vaccine for chronic hepatitis B infection, which comprises mRNA encoding the large protein (L protein) region of hepatitis B virus surface antigen, together with two mRNAs respectively encoding the middle protein (M protein) region and the small protein (S protein) region of hepatitis B virus surface antigen, or comprises mRNA encoding the small protein (S protein) region. The mRNA vaccine of the present invention may be supplemented with one or more mRNA molecules encoding different immunostimulatory factors to further activate B cells and enhance humoral immune responses for the treatment of chronic hepatitis B infection. The therapeutic mRNA vaccine of the present invention can elicit robust antiviral immune responses in mice, and is the first developed mRNA vaccine capable of breaking immune tolerance in chronic hepatitis B infection without combined administration and additional adjuvants. The vaccine of the present invention can significantly improve viral serological indicators in HBV transgenic mice and pAAV HBV1.2 mice, and has the potential to achieve functional cure of chronic hepatitis B infection in clinical practice. The present invention has been accomplished on this basis.

### Terms

For a better understanding of the present invention, certain technical and scientific terms are specifically defined below. Unless otherwise explicitly defined herein, all other technical and scientific terms used herein possess the meanings commonly understood by a skilled person in the art to which the present invention pertains. Before describing the present invention, it should be appreciated that the present invention is not limited to the specific methods and experimental conditions described, as such methods and conditions may vary. It should also be understood that the terminology used herein is for the purpose of describing particular embodiments only and is not intended to be limiting, and the scope of the present invention shall be defined solely by the appended claims.

As used herein, when employed in reference to a specifically enumerated numerical value, the term "about" means that the value may vary by no more than 1% from the recited value. For example, as used herein, the expression "about 100" encompasses all values between 99 and 101 (e.g., 99.1, 99.2, 99.3, 99.4, and the like).

As used herein, the terms "comprise", "include" and "contain" are used interchangeably and encompass not only closed definitions but also semi-closed and open-ended definitions. In other words, such terms cover "consisting of" and "consisting essentially of".

As used herein, the component referred to by the term "pharmaceutically acceptable carrier" means a substance suitable for use in humans and/or animals without undue adverse side effects (such as toxicity, irritation and allergic reaction), i.e., a substance possessing a reasonable benefit/risk ratio.

As used herein, the term "therapeutically effective amount" refers to an amount that elicits a function or activity in humans and/or animals and is tolerable by humans and/or animals. A skilled person will understand that said "therapeutically effective amount" may vary depending on factors, such as the form of the vaccine composition, administration route, excipients of the adopted medicament, severity of the disease, and combined administration with other medicaments.

### HBV surface antigen

The HBV virus possesses three surface antigens, namely the large surface antigen protein region (L protein), the middle surface antigen protein region (M protein) and the small surface antigen protein region (S protein), all encoded by the same variable open reading frame. Structurally, the S protein serves as the C-terminus region, extending the N-terminus with the Pre-S2 sequence to form the M protein, and further extending the N-terminus with the Pre-S1 sequence to form the L protein. Pre-S1 and Pre-S2 together form Pre-S.

During viral infection, the three surface antigens are synthesized in the endoplasmic reticulum and mature in the Golgi apparatus. Both the S protein and Pre-S2 can undergo glycosylation modification, and each contains a transmembrane domain at the C-terminus. Functionally, the S protein serves as the major component of surface antigens on virus and virus-like particles (VLPs), and provides structural support and facilitates nucleocapsid assembly and viral egress. The S protein binds to heparan sulfate on hepatocyte surfaces via the a-antigenic determinant, triggering amidation of the Pre-S1 domain of the L protein. Amidated Pre-S1 binds to the sodium taurocholate cotransporting polypeptide (NTCP) receptor to facilitate viral entry.

Inhibiting the binding between Pre-S1 on the surface of viral particles and the NTCP receptor can effectively block viral infection and entry. Antibody levels targeting Pre-S1 are positively correlated with a high cure rate of CHB and negatively correlated with covalently closed circular DNA (cccDNA) levels. Accordingly, designing Pre-S carrying native antigenic epitopes to activate specific humoral immune responses against the Pre-S structure represents a key strategy for mRNA vaccine design.

### mRNA vaccine

As used herein, the term "mRNA" refers to a type of single-stranded ribonucleic acid transcribed using one strand of DNA as a template, which carries genetic information and can direct protein synthesis.

As used herein, the term "vaccine" refers to a composition suitable for being administered to animals (including humans) that induces an immune response upon the administration with sufficient magnitude to minimally facilitate the prevention, improvement or cure of clinical diseases caused by viral infection.

As used herein, the terms "vaccine composition of the present invention" and "mRNA vaccine of the present invention" are interchangeable and both refer to the vaccine composition described in the first aspect of the present application.

The present invention further provides a vaccine composition comprising the mRNA vaccine of the present invention. In a preferred embodiment, the vaccine composition of the present invention is provided in the form of an mRNA-LNP vaccine.

As used herein, the term "lipid nanoparticles (LNPs)" refers to particles with at least one dimension on the nanoscale, which comprise at least one lipid. In preferred embodiments, the lipids include, but are not limited to, neutral phospholipids and polyethylene glycol-lipids. As used herein, the term "neutral phospholipid" refers to an uncharged phospholipid molecule that is not a phosphoglyceride. As used herein, the term "polyethylene glycol-lipid" or "PEG-modified lipid" refers to a molecule comprising a lipid moiety and a polyethylene glycol moiety.

In the present invention, it is confirmed by studies that the L protein can be stably expressed on the cell membrane surface and present the Pre-S structure only with the support of the M protein and the S protein (at least the S protein). Therefore, the combination of the L protein, M protein and S protein is used as the active ingredient of the vaccine to stimulate the body to produce neutralizing antibodies.

mRNA vaccines have the advantages of rapid preparation, low production costs and the ability to activate innate immune responses. They enable endogenous expression and processing of target antigens, which are then presented on the cell membrane surface, with no risk of genomic integration. After being expressed for a period of time and exerting immune effects, mRNA gradually degrades and will not be permanently expressed in cells. After multiple mRNA immunizations, the body can mount a sufficiently strong memory response targeting specific antigens.

In the present invention, mRNA molecules encoding the L protein, M protein and S protein are designed, which are separately encapsulated by LNPs. After a mixture of L mRNA, M mRNA and S mRNA, or a mixture of L mRNA and S mRNA are used for the transfection, a relatively high proportion of L protein is displayed on the cell membrane surface with the support of the M protein and S protein or only the S protein, so that B cells can be activated either through direct presentation or by presenting native Pre-S antigen epitopes via MHC molecules. In HBV Tg mice with complete immune tolerance, the production of antibodies targeting native Pre-S antigen epitopes as well as HBsAbs can be significantly stimulated, thereby effectively breaking immune tolerance and eliminating surface antigens. Meanwhile, mRNA of immunostimulatory factors represented by CD40L is designed and prepared, which, after co-transfection and expression, can bind to CD40 on the surface of B cells and serve as a second signal to enhance B cell activation, thereby further boosting humoral immune responses.

The vaccine composition provided in the present invention can be in any suitable form depending on the administration method required by the patient, which may be provided in unit dosage form, usually placed in a sealed container, and may be provided as part of a kit. Such kits generally (but not necessarily) contain instructions and may comprise a plurality of the unit dosage forms.

The vaccine composition is suitable for any appropriate administration route, such as injection (including subcutaneous, intradermal, intramuscular, intraperitoneal, microneedle or intravenous injection), inhalation, oral administration, nasal administration, or rectal administration. The composition can be prepared by any method known in the pharmaceutical field, for example, by mixing the active ingredient with a carrier or excipient under sterile conditions.

### HBV mRNA vaccine

The presentation of natural antigen epitopes and stable antigen expression are critical for HBV mRNA vaccines. Meanwhile, mRNA sequences markedly affect their stability and translation efficiency. Therefore, in the present invention, in addition to retaining the native amino acid sequences of L protein, M protein and S protein, the gene coding sequences (CDS sequences) are optimized respectively, untranslated regions 5'-UTR and 3'-UTR are added before and after the CDS sequences and poly adenylic acid (polyA) is added, based on which plasmid templates constructed for *in vitro* transcription significantly improves mRNA stability and translation efficiency. The transcription templates also contain functional elements, such as T7 promoter and prokaryotic expression resistance genes. During *in vitro* transcription, 5' capping of mRNA and incorporation of modified nucleosides are performed. After mRNA purification and quality control, cationic LNPs are used for encapsulation, followed by quality testing of LNP/mRNA.

The specific amino acid sequences are shown as follows:
L protein (SEQ ID NO: 1) 
MGGWSSKPRKGMGTNLSVPNPLGFFPDHQLDPAFKANSENPDWDLNP
HKDNWPDANKVGVGAFGPGFTPPHGGLLGWSPQAQGLLTTVPAAPPPASTN
RQSGRQPTPLSPPLRDTHPQAMQWNSTTFHQTLQDPRVRALYFPAGGSSSGT
VSPAQNTVSAISSILSKTGDPVPNMENIASGLLGPLLVLQAGFFLLTKILTIPQS
LDSWWTSLNFLGGTPVCLGQNSQSQISSHSPTCCPPICPGYRWMCLRRFIIFLC
ILLLCLIFLLVLLDYQGMLPVCPLIPGSSTTSTGPCKTCTTPAQGTSMFPSCCC
TKPTDGNCTCIPIPSSWAFAKYLWEWASVRFSWLSLLVPFVQWFVGLSPTVW
LSVIWMMWFWGPSLYNILSPFMPLLPIFFCLWVYI
M protein (SEQ ID NO: 2) 
MQWNSTTFHQTLQDPRVRALYFPAGGSSSGTVSPAQNTVSAISSILSKT
GDPVPNMENIASGLLGPLLVLQAGFFLLTKILTIPQSLDSWWTSLNFLGGTPV
CLGQNSQSQISSHSPTCCPPICPGYRWMCLRRFIIFLCILLLCLIFLLVLLDYQG
MLPVCPLIPGSSTTSTGPCKTCTTPAQGTSMFPSCCCTKPTDGNCTCIPIPSSW
AFAKYLWEWASVRFSWLSLLVPFVQWFVGLSPTVWLSVIWMMWFWGPSL
YNILSPFMPLLPIFFCLWVYI
S protein (SEQ ID NO: 3)

MENIASGLLGPLLVLQAGFFLLTKILTIPQSLDSWWTSLNFLGGTPVCLG
QNSQSQISSHSPTCCPPICPGYRWMCLRRFIIFLCILLLCLIFLLVLLDYQGMLP
VCPLIPGSSTTSTGPCKTCTTPAQGTSMFPSCCCTKPTDGNCTCIPIPSSWAFA
KYLWEWASVRFSWLSLLVPFVQWFVGLSPTVWLSVIWMMWFWGPSLYNIL
SPFMPLLPIFFCLWVYI

In the present invention, an mRNA vaccine is provided, which encodes the hepatitis B surface antigen large protein (L protein), hepatitis B surface antigen middle protein (M protein) and hepatitis B surface antigen small protein (S protein), or encodes the L protein and the S protein, and exerts therapeutic effects as a therapeutic vaccine in HBV infection or chronic hepatitis B infection. The vaccine of the present invention may comprise the mRNA of the present invention, or a complex formed by the mRNA and lipid nanoparticle (LNP). The vaccine of the present invention can induce massive production of antibodies against the S protein in a chronic hepatitis B infection model, break immune tolerance in chronic HBV infection, and significantly reduce serum HBsAg levels. The vaccine of the present invention can achieve partial functional cure in a chronic hepatitis B infection model.

In the present invention, mRNAs encoding L protein, M protein and S protein are respectively designed, optimized and prepared as components of the aforementioned mRNA vaccine, and the coding sequences are shown in SEQ ID NO: 4-6.

In an embodiment, a therapeutic mRNA vaccine for chronic hepatitis B infection is provided, which comprises L mRNA, M mRNA and S mRNA, or L mRNA and S mRNA. Different mRNAs are separately encapsulated by LNPs and then mixed for use.

In an embodiment, mammalian cells are transfected with the combination of L mRNA, M mRNA and S mRNA, or the combination of L mRNA and S mRNA. The expression of L protein, M protein and S protein can be detected on cell membranes and in cell culture supernatants.

Immunizing HBV Tg mice with complete immune tolerance using the mRNA vaccine of the present invention can stimulate high-level production of HBsAb, break immune tolerance in CHB, significantly reduce HBsAg levels in some mice and improve viral serological indicators, indicating that this mRNA vaccine has the potential to achieve functional cure of CHB.

After immunizing HBV Tg mice with the mRNA vaccine of the present invention, the antibody IgGs in mouse serum are purified and anti-S IgG antibodies are removed. The remaining IgG can bind to HBV virions, demonstrating that the vaccine can induce the production of specific antibodies targeting native Pre-S antigen epitopes.

After immunizing HBV Tg mice with the mRNA vaccine of the present invention and upon completion of the treatment, splenic T cells are isolated. Stimulation of T cells with mouse cells co-expressing L protein, M protein and S protein induces IFN-γ expression, indicating that the mRNA vaccine specifically elicited T cell immune responses against Pre-S antigen epitopes.

Immunization of the pAAV HBV1.2 mouse model with the mRNA vaccine of the present invention can trigger HBsAb production, significantly reduce HBsAg and HBeAg levels in some mice, and improve viral serological indicators.

Immunization of the rAAV HBV1.3 mouse model with the mRNA vaccine of the present invention can trigger HBsAb production, significantly reduce HBsAg and HBeAg levels in some mice, and improve viral serological indicators.

In one embodiment, the mRNA vaccine of the present invention can be administered together with an immunostimulatory factor (e.g., CD40L).

The mRNA encoding CD40L designed and prepared in the present invention can significantly activate B cells after transfecting mammalian cells and incubating with PBMCs or B cells isolated from mouse peripheral blood.

When the mRNA encoding CD40L designed and prepared by the present invention is co-immunized with the mRNA vaccine into HBV Tg mice, it can enhance the humoral immune response by the mRNA vaccine, elevate the level of antibodies and markedly reduce the surface antigen level.

The present invention also provides a method capable of inducing the production of antibodies targeting native Pre-S antigen epitopes and maintaining such antibody production for a long duration in a chronic hepatitis B infection model, which comprises administering the mRNA vaccine of the present invention to a subject in need thereof.

Main advantages of the present invention include:
1) After transfecting cells with the mRNA vaccine of the present invention, the co-expression of L protein, S protein and optional M protein enables stable expression of hepatitis B surface antigen on the cell surface and presentation of Pre-S epitopes, which solves the problem that L protein cannot be stably expressed on the cell surface alone.
2) The mRNA vaccine of the present invention exhibits advantages, such as simple preparation process, short production cycle, low cost and favorable immune effect, and can significantly stimulate the generation of high-level antibodies.
3) The mRNA vaccine of the present invention can break immune tolerance in HBV Tg models, induce the production of specific antibodies targeting the native Pre-S antigen structure and elicit Pre-S-dependent T cell immune responses.

The present invention will be further described below in conjunction with specific examples. It should be understood that these examples are merely intended to illustrate the present invention rather than limit its scope. Experimental methods without specific conditions specified in the following examples are generally carried out under conventional conditions, such as those described in Sambrook et al., Molecular Cloning: A Laboratory Manual (New York: Cold Spring Harbor Laboratory Press, 1989), or under the conditions recommended by manufacturers. Unless otherwise specified, all percentages and parts refer to weight percentages and parts by weight.

### Example 1 Expression and Detection of Cell Membrane Protein L

First, L protein, M protein and S protein expression plasmids (all pD2531 plasmids) were used to stably transfect glutamine synthetase-deficient (Gln⁻) 293 cells. The main procedures are as follows:
(1) The three plasmids were linearized with PvuI-HF enzyme (NEB);
(2) PEI Prime (Serochem) was mixed with linearized plasmids and 293 (Gln⁻) cells were transfected in shake flasks. Experimental groups include pD2531: L + pD2531:S and pD2531:L + pD2531:M + pD2531:S, with untransfected 293 (Gln⁻) cells as control;
(3) One week after transfection, when cell viability and proliferation were favorable, transfected cells were continuously cultured in glutamine-deficient 293 medium (Gibco). Samples were collected two weeks later, and antigen expression was detected via flow cytometry (FACS);
(4) The selection and culture of monoclonal cells were conducted by limiting dilution. Finally, monoclonal cell lines 293:LS(4C6) and 293:LS(4G2) expressing L protein and S protein, as well as monoclonal cell line 293:LMS(A4) expressing L protein, M protein and S protein were obtained respectively.

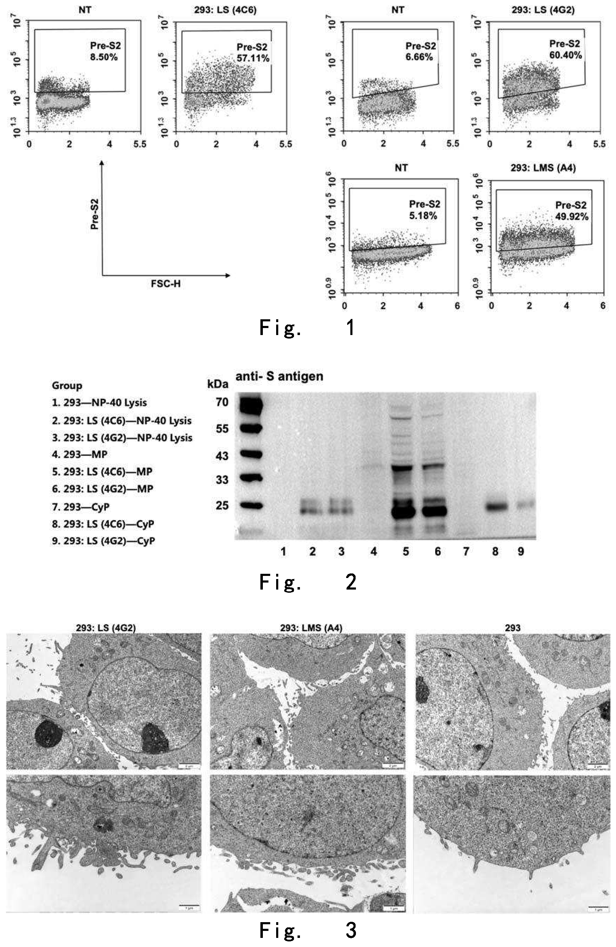

After multiple passages, FACS analysis revealed that the positive rate of Pre-S2 antigen on the surface of the aforementioned monoclonal cells ranged from 45% to 55% (Figure 1). A Membrane Protein Extraction Kit (Beyotime) was used to extract membrane proteins (MP) and cytoplasmic proteins (CyP) from 293, 293:LS(4C6) and 293:LS(4G2) cells. The main procedures included hypotonic treatment of cells, 2-3 cycles of freeze-thawing in liquid nitrogen, low-speed centrifugation to precipitate cell nuclei, high-speed centrifugation to harvest membrane fractions, and dissolution of membrane proteins. Total cellular proteins lysed with NP-40 lysis buffer were used as controls. Western blotting (WB) was performed to detect the S antigen region in each fraction. As shown in Figure 2, certain proportions of S protein and L protein were detected in the membrane fractions (at approximately 40 kDa molecular weight).

All the above results indicate that L protein can be stably displayed on the cell membrane surface with the support of S protein or M protein plus S protein (Figures 1, 2, 16, 17, 18).

Meanwhile, consistent with other studies, when 293 cells were stably transfected with the pD2531:L plasmid alone, most cells died due to cytotoxicity during screening, and single clones could not be isolated.

### Example 2 Observation of cell membrane raft structure

Membrane raft is a dynamic lipid raft structure protruding on the cell membrane surface, which contains lipids, proteins and sugar molecules. Membrane rafts play a certain role in the invasion, assembly and budding of viruses. The expression of viral surface antigens can sometimes induce the formation of such structures on the membrane surface, which present antigen molecules arranged regularly at a certain density. This exerts a strong promoting effect on enhancing the immunogenicity of antigens. Therefore, in this Example, transmission electron microscopy (TEM) was used to observe the distribution of membrane rafts on the surface of 293:LS(4G2) cells and 293:LMS(A4) cells, with 293(Gln-) cells set as the control. The main procedures were as follows: cells were cultured in shake flasks for a period of time and then harvested by centrifugation, fixed with 2.5% glutaraldehyde, and then delivered to a third-party testing institution for TEM sample preparation and imaging. The results are shown in Figure 3. Compared with the control cells, abundant membrane raft structures were observed on the surface of both monoclonal cells. This indicates that the co-expression of S protein and L protein, or S protein, M protein and L protein can drive the formation of membrane rafts (Figure 3), and the L protein presented on the cell membrane may possess strong immunogenicity.

### Example 3 Preparation of mRNA Vaccine

According to the results of Example 1, the L protein can be stably presented on the cell membrane only when the L protein and S protein are co-expressed, or the L protein, M protein and S protein are co-expressed. mRNAs encoding these three surface antigen proteins were separately designed, optimized and prepared. Lipid nanoparticle (LNP) was used as the delivery vector, and intramuscular injection of mRNA vaccine was adopted as the immunization route. *In vitro* experiments verified that these three proteins can be efficiently expressed in mammalian cells.

The preparation method for mRNA vaccines mainly includes: the optimized CDS sequences of L protein, M protein and S protein were separately synthesized, the DNA fragments were cloned into template plasmids respectively, mRNAs were prepared via *in vitro* transcription and purified, followed by LNP encapsulation and quality control testing.

The optimization of CDS sequences includes adjusting codon bias for expression in human cells, increasing GC content, tuning the usage frequency of commonly used codons, avoiding specific restriction enzyme sites, maintaining high structural stability of mRNA, and achieving higher translation efficiency in humans and mammals. The transcription start sites of the M protein and S protein within the sequence were mutated, so that they can only be transcribed and translated into the L protein. Additionally, the transcription start site of the S protein located in the M protein sequence was mutated to ensure exclusive translation of the M protein.

The mRNA sequences are shown below:
L mRNA (SEQ ID NO: 4): 
AUGGGCGGAUGGAGCAGCAAGCCCAGGAAGGGCAUGGGCACCAAC
CUGAGCGUGCCCAAUCCCCUGGGCUUCUUCCCCGACCACCAGCUGGACC
CCGCCUUUAAGGCCAACAGCGAGAACCCCGACUGGGACCUGAAUCCCCA
CAAGGAUAACUGGCCCGACGCCAACAAAGUGGGAGUCGGCGCCUUCGG
ACCCGGCUUCACACCUCCUCACGGAGGACUCCUGGGAUGGUCCCCCAA
GCCCAGGGACUGCUGACCACAGUGCCCGCUGCUCCUCCUCCCGCUAGCA
CCAAUAGGCAGUCCGGCAGGCAACCUACCCCCCUGAGCCCCCCUCUGAG
GGAUACACACCCUCAGGCCAUGCAGUGGAACUCCACCACCUUCCAUCAG
ACCCUGCAAGAUCCUAGGGUGAGGGCCCUGUACUUCCCUGCCGGAGGC
AGCUCCAGCGGAACAGUGAGCCCCGCUCAGAACACAGUGAGCGCCAUC
AGCAGCAUCCUGUCCAAGACCGGCGACCCCGUGCCCAAUAUGGAAAAU
AUCGCCAGCGGCCUGCUGGGACCCCUGCUGGUGCUGCAGGCCGGCUUCU
UCCUGCUGACCAAAAUCCUGACCAUCCCCCAGUCCCUGGAUUCCUGGUG
GACCUCCCUGAACUUCCUGGGCGGCACCCCUGUGUGUCUCGGCCAGAAC
UCCCAGUCCCAGAUCUCCUCCCACAGCCCUACCUGCUGCCCCCCUAUCU
GCCCCGGCUAUAGAUGGAUGUGCCUGAGGAGGUUCAUCAUCUUUCUGU
GCAUCCUGCUGCUGUGCCUGAUCUUUCUCCUGGUGCUCCUCGACUACCA
GGGCAUGCUGCCCGUGUGUCCUCUGAUCCCCGGCAGCAGCACCACCUCC
ACAGGCCCUUGCAAAACCUGCACCACACCCGCCCAGGGCACAAGCAUGU
UCCCUUCCUGCUGCUGCACCAAGCCCACCGACGGCAACUGCACCUGCAU
CCCCAUUCCCAGCAGCUGGGCCUUCGCCAAGUAUCUGUGGGAGUGGGC
CAGCGUGAGGUUCAGCUGGCUGAGCCUGCUCGUGCCCUUCGUGCAGUG
GUUCGUCGGCCUGAGCCCUACCGUGUGGCUGUCCGUGAUCUGGAUGAU
GUGGUUCUGGGGCCCCAGCCUGUACAAUAUCCUGUCCCCCUUCAUGCCC
CUGCUGCCUAUCUUCUUCUGCCUGUGGGUCUACAUCUGA
M mRNA (SEQ ID NO: 5) 
AUGCAGUGGAACUCCACCACCUUCCAUCAGACCCUGCAAGAUCCUA
GGGUGAGGGCCCUGUACUUCCCUGCCGGAGGCAGCUCCAGCGGAACAG
UGAGCCCCGCUCAGAACACAGUGAGCGCCAUCAGCAGCAUCCUGUCCAA
GACCGGCGACCCCGUGCCCAAUAUGGAAAAUCGCCAGCGGCCUGCU
GGGACCCCUGCUGGUGCUGCAGGCCGGCUUCUUCCUGCUGACCAAAAU
CCUGACCAUCCCCCAGUCCCUGGAUUCCUGGUGGACCUCCCUGAACUUC
CUGGGCGGCACCCCUGUGUGUCUCGGCCAGAACUCCCAGUCCCAGAUCU
CCUCCCACAGCCCUACCUGCUGCCCCCCUAUCUGCCCCGGCUAUAGAUG
 
GAUGUGCCUGAGGAGGUUCAUCAUCUUUCUGUGCAUCCUGCUGCUGUG
CCUGAUCUUUCUCCUGGUGCUCCUCGACUACCAGGGCAUGCUGCCCGUG
UGUCCUCUGAUCCCCGGCAGCAGCACCACCUCCACAGGCCCUUGCAAAA
CCUGCACCACACCCGCCCAGGGCACAAGCAUGUUCCCUUCCUGCUGCUG
CACCAAGCCCACCGACGGCAACUGCACCUGCAUCCCCAUUCCCAGCAGC
UGGGCCUUCGCCAAGUAUCUGUGGGAGUGGGCCAGCGUGAGGUUCAGC
UGGCUGAGCCUGCUCGUGCCCUUCGUGCAGUGGUUCGUCGGCCUGAGC
CCUACCGUGUGGCUGUCCGUGAUCUGGAUGAUGUGGUUCUGGGGCCCC
AGCCUGUACAAUAUCCUGUCCCCCUUCAUGCCCCUGCUGCCUAUCUUCU
UCUGCCUGUGGGUCUACAUCUGA
S mRNA (SEQ ID NO: 6)

AUGGAAAAUAUCGCCAGCGGCCUGCUGGGACCCCUGCUGGUGCUG
CAGGCCGGCUUCUUCCUGCUGACCAAAAUCCUGACCAUCCCCCAGUCCC
UGGAUUCCUGGUGGACCUCCCUGAACUUCCUGGGCGGCACCCCUGUGU
GUCUCGGCCAGAACUCCCAGUCCCAGAUCUCCUCCCACAGCCCUACCUG
CUGCCCCCCUAUCUGCCCCGGCUAUAGAUGGAUGUGCCUGAGGAGGUU
CAUCAUCUUUCUGUGCAUCCUGCUGCUGUGCCUGAUCUUUCUCCUGGU
GCUCCUCGACUACCAGGGCAUGCUGCCCGUGUGUCCUCUGAUCCCCGGC
AGCAGCACCACCUCCACAGGCCCUUGCAAAACCUGCACCACACCCGCCC
AGGGCACAAGCAUGUUCCCUUCCUGCUGCUGCACCAAGCCCACCGACGG
CAACUGCACCUGCAUCCCCAUUCCCAGCAGCUGGGCCUUCGCCAAGUAU
CUGUGGGAGUGGGCCAGCGUGAGGUUCAGCUGGCUGAGCCUGCUCGUG
CCCUUCGUGCAGUGGUUCGUCGGCCUGAGCCCUACCGUGUGGCUGUCC
GUGAUCUGGAUGAUGUGGUUCUGGGGCCCCAGCCUGUACAAUAUCCUG
UCCCCCUUCAUGCCCCUGCUGCCUAUCUUCUUCUGCCUGUGGGUCUACA
UCUGA

The transcription template plasmid contains elements including T7 promoter, 5'-UTR, coding region (ORF), 3'-UTR, polyA, kanamycin resistance sequence (KanR), and the like.

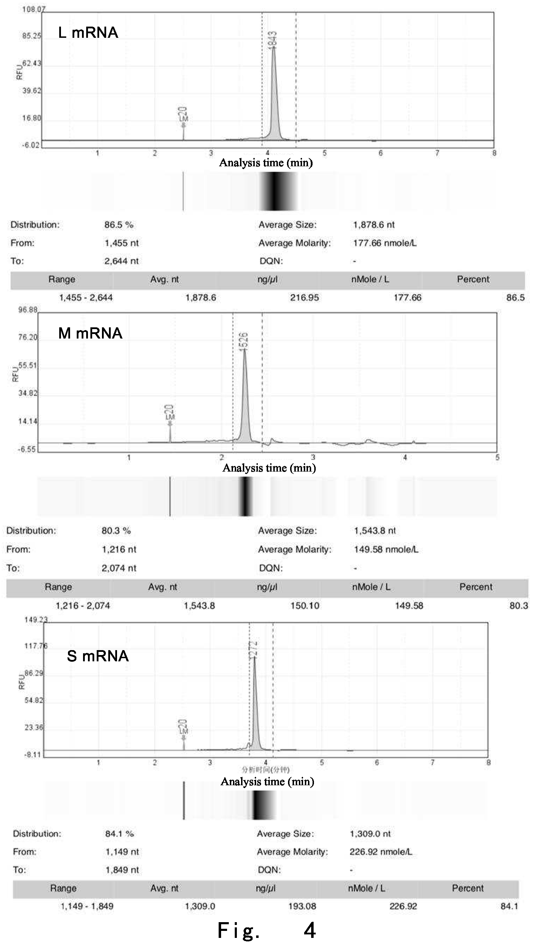

mRNA can be prepared by *in vitro* transcription via a three-step, two-step or one-step method, yielding mRNA molecules with 5' capping of Cap1 or Cap0 type, 3' polyadenylation, and incorporation of pseudouridine and/or other modified nucleosides. DNase I was applied to remove DNA templates, and lithium chloride was used for mRNA purification. After reconstitution with RNase Free Water, the concentration and purity of mRNA were determined by NanoDrop ultraviolet spectrophotometer, the length was verified by agarose gel electrophoresis, the capping efficiency and poly(A) tail status were analyzed by LC-MS, and mRNA integrity was assessed by capillary electrophoresis (Figure 4).

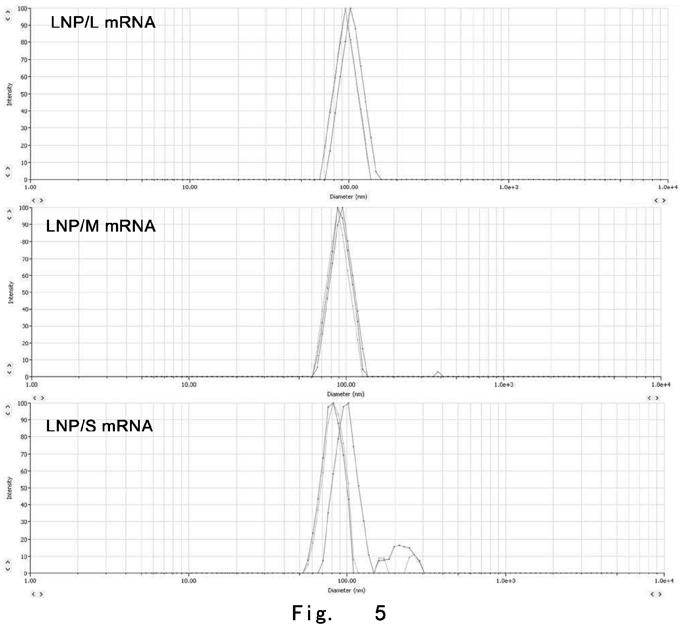

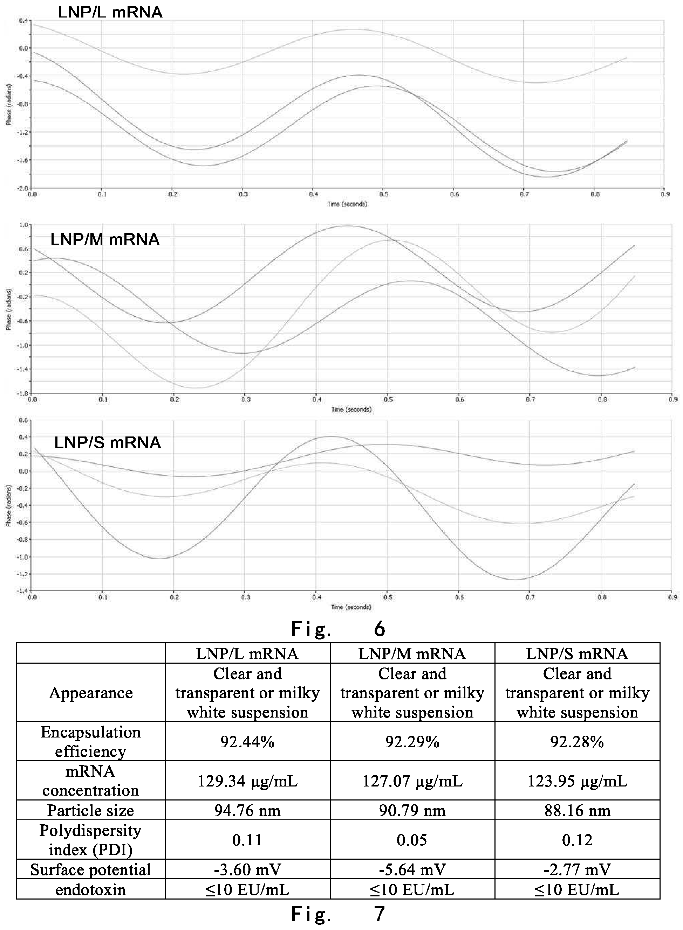

In the present invention, lipid nanoparticle (LNP) was used to encapsulate mRNA. The LNP comprises four components, namely cationic lipid SM-102 (50%), helper lipid DSPC (10%), cholesterol (Chol, 38.5%), and PEG2000-DMG lipid (1.5%). These four components were dissolved in anhydrous ethanol according to molar ratios to form an organic phase. The mRNA contained in the aqueous phase and the organic phase were injected into a microfluidic device at a specific ratio and mixed to obtain an LNP/mRNA mixture. The final LNP/mRNA product was obtained via buffer exchange, sterile filtration and concentration. Nanosight was used to determine particle size (results shown in Figure 5) and polydispersity index (PDI); phase analysis light scattering was applied to measure surface potential (results shown in Figure 6); RiboGreen assay was utilized to test encapsulation efficiency and mRNA concentration; limulus amebocyte lysate test was adopted to detect the bacterial endotoxin level of LNP/mRNA. The summarized results are presented in Figure 7. The product was stored at 4 °C until use.

All injected LNP/mRNA of the present invention were used within one month after encapsulation.

### Example 4 In Vitro Expression and Verification of mRNA Vaccine

293T cells were inoculated into 6-well plates at a density of 6×10⁵ cells per well. After overnight incubation, each well received no treatment or is supplemented with 5 µg of LNP-encapsulated L mRNA, M mRNA, S mRNA, 5 µg L mRNA + 5 µg S mRNA, or 5 µg L mRNA + 5 µg M mRNA + 5 µg S mRNA, respectively. At 48 hours post-transfection, cells were harvested and split into two aliquots. One aliquot was subjected to FACS analysis, while the other was used for membrane protein extraction, and all the aliquots along with cell culture supernatants were subjected to WB analysis. The identical transfection procedure was performed, followed by FACS analysis at 96 hours post-transfection. Details are as follows:
(1) FACS detection: cells of each group were washed once with pre-cooled PBS. After centrifugation, the cells were blocked with 3% BSA on ice for 30 min. The cells were split into two aliquots. Following centrifugation, diluted anti-Pre-S2 antibody and anti-S antibody were added to the two aliquots respectively, and incubated on ice for 1 h. After washing three times with PBS, FITC anti-mouse secondary antibody and FITC anti-rabbit secondary antibody were added separately, incubated on ice for 0.5 h, washed three times with PBS, and subjected to flow cytometry analysis.
(2) Membrane protein extraction and WB detection: The membrane protein extraction method was the same as that in Example 1, mainly including cell hypotonic treatment, 2-3 cycles of liquid nitrogen freeze-thawing, low-speed centrifugation to precipitate cell nuclei, high-speed centrifugation to precipitate membrane components, and dissolution of membrane proteins. 5×loading buffer was added to cell membrane lysate and cell culture supernatant, followed by incubation in boiling water bath incubation for 5 min. Subsequently, polyacrylamide gel electrophoresis was performed. After electrophoresis, Western blotting was carried out, and proteins were to transferred onto PVDF membranes. The membranes were blocked with 5% skim milk powder at room temperature for 1 h, incubated overnight at 4°C with anti-Pre-S2 and anti-S antibodies respectively, and washed 3 times with TBST. The membranes were incubated with HRP anti-mouse antibody at room temperature for 1 h and washed 3 times with TBST. Enhanced chemiluminescence reagent was used for color development and exposure of PVDF membranes, followed by photographing.

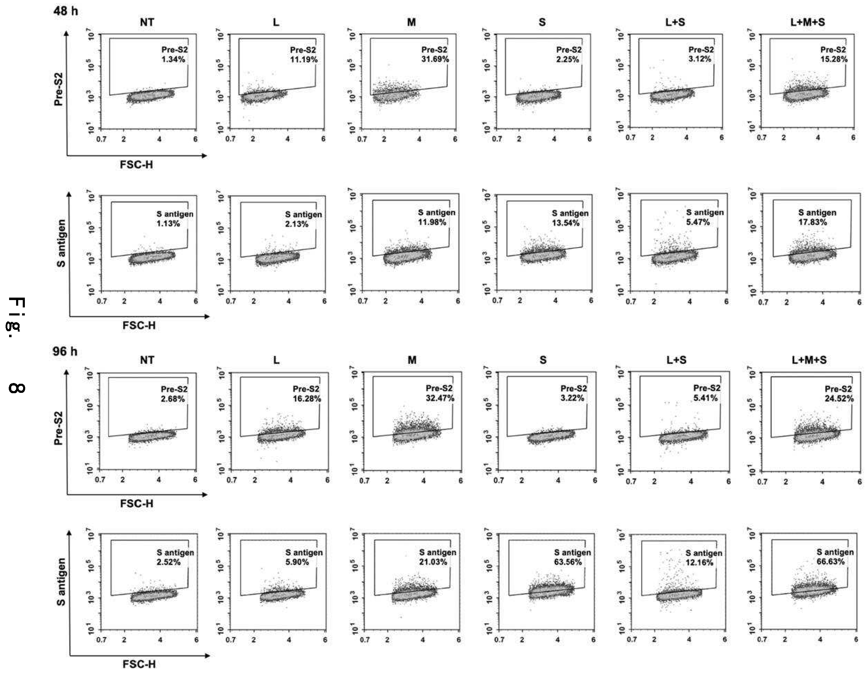

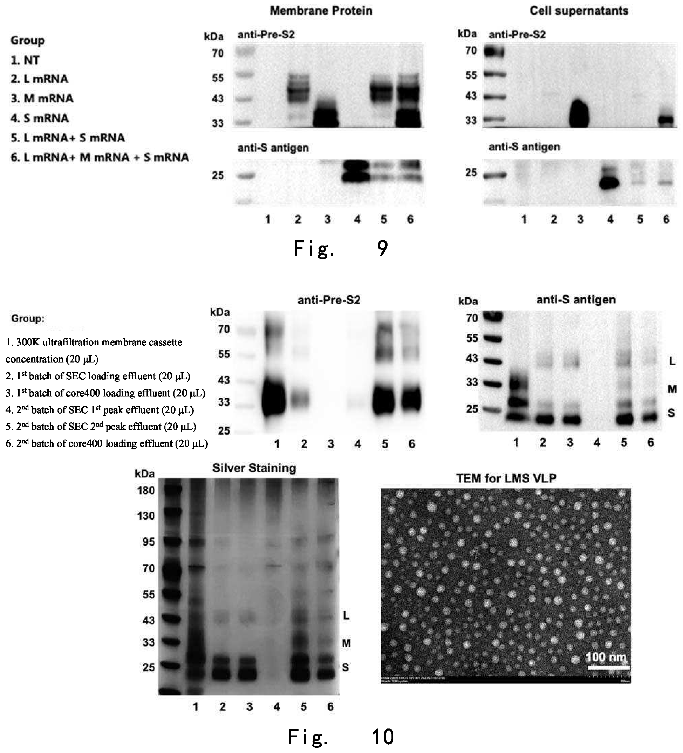

The FACS detection results are shown in Figure 8. Except that the Pre-S2 signal was the highest in the M mRNA transfection group, the signals of Pre-S2 and S proteins reached the highest levels upon co-transduction of the three mRNAs compared with other groups, indicating a higher proportion of corresponding antigens presented on the cell membrane surface. The WB detection results are presented in Figure 9. Under the condition of co-transduction of the three mRNAs, the cell membrane could present all three proteins. Compared with separate transfection of L mRNA, the expression level and proportion of L protein were significantly increased. In addition, S protein and M protein could be detected in the cell culture supernatant during co-transduction.

### Example 5 Study on mRNA Vaccine Breaking Immune Tolerance in HBV Transgenic Mice

The activation of hepatitis B surface antibodies by mRNA vaccines under the immune tolerant state of chronic hepatitis B was investigate in 1.3-copy HBV transgenic Tg Balb/c mice and the correlation between antigen levels and antibody production levels was explored. After breeding of 1.3-copy HBV Tg mice, the positive rate of offspring mice carrying HBV was 75%, and the mice expressed and carried HBV virions lifelong from birth. The serum hepatitis B surface antigen (HBsAg) of transgenic mice was detected by Abbott chemiluminescence assay which reached 15517.07 ± 9376.81 IU/mL. The serum HBV DNA was detected by Sansure HBV nucleic acid quantitative detection kit (PCR-fluorescent probe method) and ranged from 10³ to 10⁵ IU/mL. No significant gender-related differences were observed in HBsAg and HBV DNA levels of mice. The level of hepatitis B surface antibody (HBsAb) in all transgenic mice was 0. This transgenic mouse strain is mainly applied in researches on immune pathogenesis of hepatitis B and efficacy evaluation of anti-hepatitis B virus drugs, and it is the most widely used HBV transgenic mouse model in China at present.

55 HBV Tg mice with varying HBsAg levels were evenly divided into 11 groups, with 5 mice per group. Each group contained mice with high HBsAg levels (>10000 IU/mL) and low HBsAg levels (<10000 IU/mL), which were injected with different mRNA vaccines, proteins or PBS respectively. The grouping was as follows:
PBS;
Luciferase (Luc) mRNA (10 µg);
L+M+S mRNA (5+5+5 µg / 10+10+10 µg);
L+S mRNA (5+5 µg / 10+10 µg);
L mRNA (5 µg / 10 µg);
S mRNA (10 µg);
Virus-like particles LMS VLP (5 µg) + 125 µg aluminum adjuvant Alhydrogel^{®} adjuvant (InvivoGen);
Pre-S peptide (5 µg) + 125 µg Alhydrogel^{®} adjuvant.

Mice were immunized via intramuscular injection on Day 0, Day 8, Day 15, Day 29 and Day 113 (16 weeks). Orbital blood collection was performed on Day 0 and every two weeks thereafter to obtain serum until the end of the experiment. Serum HBsAg and HBsAb were detected by chemiluminescence assay using Abbott reagents, and serum HBV DNA was quantified using the Sansure HBV Nucleic Acid Quantitative Detection Kit.

Luc mRNA was obtained commercially. Pre-S protein was acquired through prokaryotic expression and purification. The specific methods include following steps:
(1) Construction of the prokaryotic expression system involves synthesizing the Pre-S gene and cloning it into the pET:28a prokaryotic expression plasmid downstream of the 6 × His tag. The plasmid carries the KanR resistance gene and isopropyl β-D-1-thiogalactopyranoside (IPTG). The constructed plasmid was transformed into BL21 (DE3) competent cells. After bacterial shaking culture and plate coating, colonies were picked following overnight incubation for colony PCR. Target bands were identified by molecular weight via agarose gel electrophoresis. Positive colonies were selected and sent to GENEWIZ for sequencing. After sequence verification, bacterial liquid amplification and strain preservation were carried out.
(2) The prokaryotic expression and purification process includes strain resuscitation, amplification using LB medium, IPTG-induced expression, bacterial liquid collection and lysis, extraction and refolding of inclusion body proteins, and purification by Ni column to obtain the target protein;
(3) Finally, endotoxin was removed using Thermo Fisher Pierce High-Capacity Endotoxin Removal Resin, and the endotoxin level was tested with GenScript endotoxin detection kit.

In this Example, LMS VLPs were obtained by fermentation and purification of the 293:LMS (A4) monoclonal cell line constructed and screened in Example 1, which secretes LMS VLP particles. The specific procedures are as follows: the 293 monoclonal cells were expanded and fermented in shake flasks ranging from 500 mL to 2.5 L; upon completion of fermentation, the cells were centrifuged at 3000 rpm/min, followed by centrifugation at 8000 rpm/min to remove cell debris. After 50-70-fold tangential flow concentration against a 300K ultrafiltration membrane cassette and buffer exchange with D-PBS, VLPs were purified via hydroxyapatite CHT adsorption, size-exclusion chromatography (SEC), and chromatography using core400 macromolecule resin for viruses/biomacromolecules. Silver staining was adopted to detect the purity of LMS VLPs and the proportion of each protein; Western blot (WB) was used to further verify protein L, protein M and protein S; and TEM was applied to detect the morphology and size of VLPs. The results are shown in Figure 10.

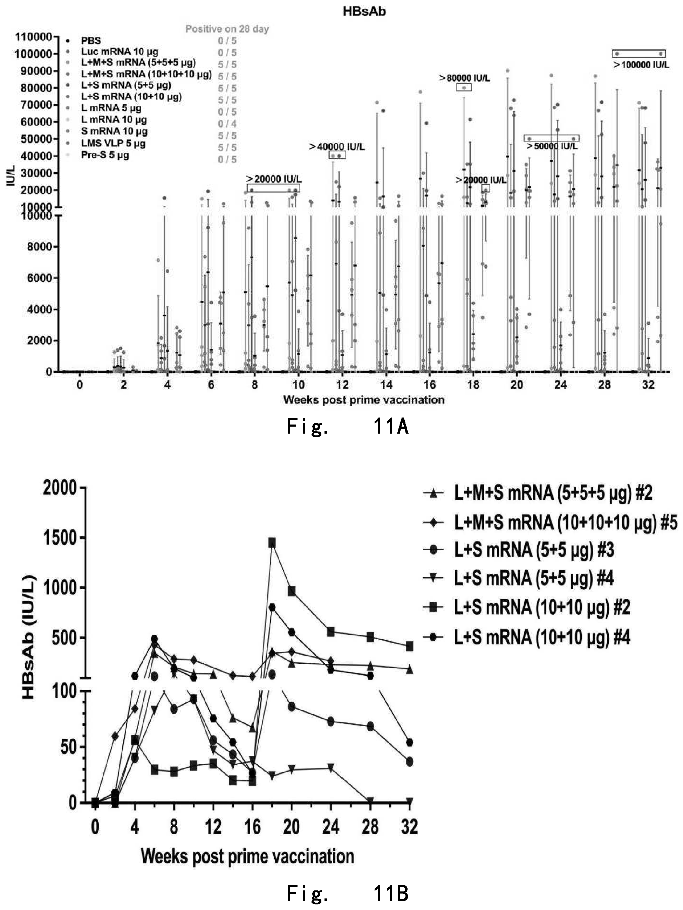

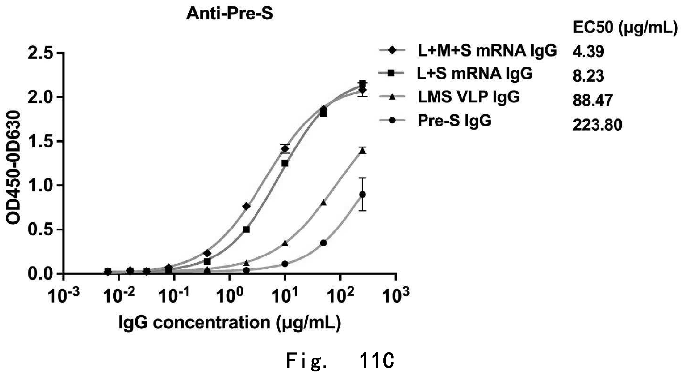

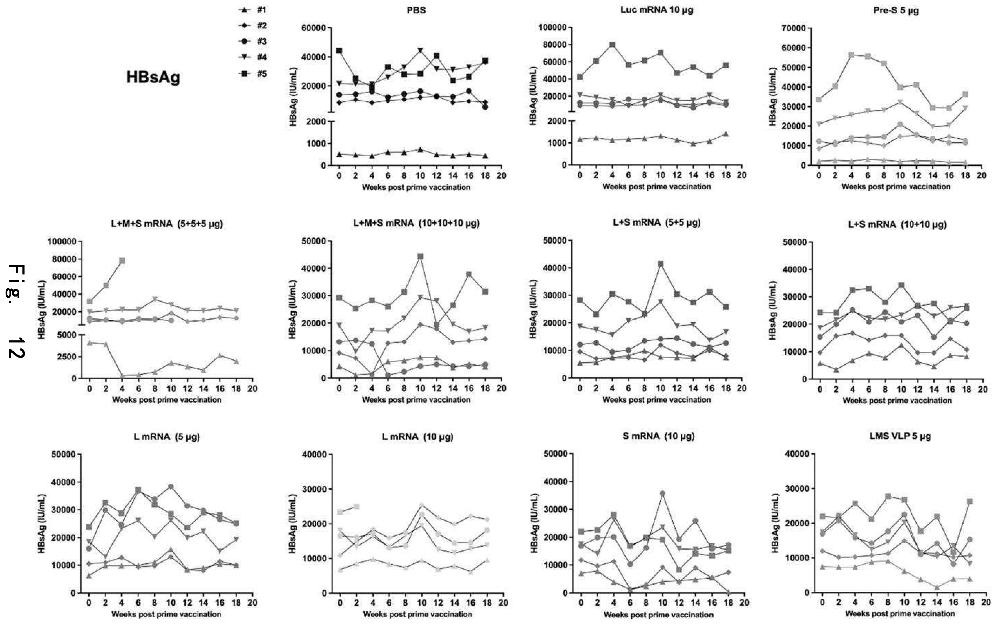

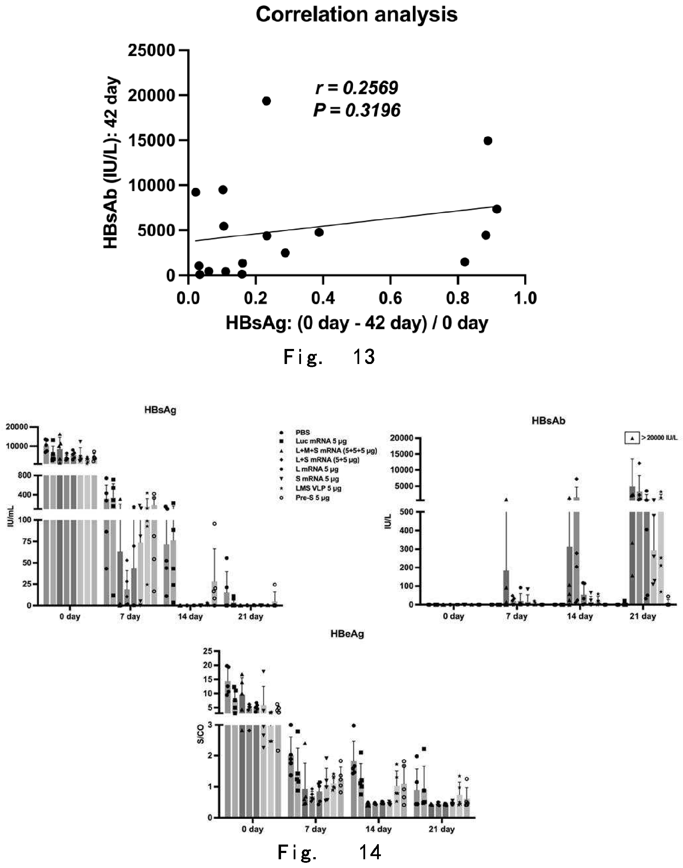

The HBsAb test results are shown in Figure 11. Two weeks after the first immunization (Week 2), all mice in the high-dose L+M+S mRNA group and 80% of mice in the low-dose L+M+S mRNA group developed surface antibodies. 80%, 60% and 60% of mice in the high- and low-dose L+S mRNA groups and the S mRNA group generated antibodies, respectively. The overall antibody level in the L+S mRNA group was higher than that in the S mRNA group. In comparison, 60% of mice in the LMS VLP group produced antibodies. Two weeks after the second immunization (Week 4), all mice in the L+M+S mRNA, L+S mRNA and S mRNA groups generated antibodies and broke immune tolerance (surface antibody level > 10 IU/L). Although all mice in the LMS VLP group generated antibodies by Week 4, immune tolerance was not completely broken until Week 6. No antibodies were detected in the L mRNA group at any time point. Meanwhile, starting from Week 4, most mice in the L+M+S mRNA, L+S mRNA, S mRNA and LMS VLP groups maintained persistently high levels of surface antibodies, especially those in the former two groups. Antibody titers above 40000 IU/L were generated starting from Week 14 and continued to rise. Antibody levels further increased two weeks after the final immunization at Week 16 (Week 18). The HBsAg test results are shown in Figure 12. From Week 0 to Week 18, one mouse with low antigen level in each of the L+M+S mRNA, S mRNA and LMS VLP groups exhibited markedly reduced surface antigen. Correlation analysis was performed between antigen reduction and antibody levels in mice which generated antibodies within Weeks 0-6, and no significant correlation was observed. This indicates that the capacity of mRNA vaccines to activate antibody and break immune tolerance will not be affected by antigen levels (Figure 13).

### Example 6 Study on the Therapeutic Effect of mRNA Vaccine in pAAV HBV1.2 Mouse Model

The pAAV HBV 1.2-copy mouse model was constructed as follows: 10 µg of pAAV HBV 1.2 plasmid was injected into C57/BL6 mice via the tail vein at a volume equivalent to 8% of the mouse body weight. This induced transient cardiac congestion and elevated downstream pressure, driving the plasmid solution toward the hepatic and renal veins. The pressure opened the barrier between endothelial cells and created transient defects in hepatocyte membranes. Mice recovered to normal status within 12 h to 72 h. The pAAV HBV 1.2 was delivered into hepatocytes to replicate and express HBV viral particles. Three days after model establishment, HBsAg and HBV DNA generally reached peak levels, and positive surface antigen can be sustained for approximately one month.

On Day 4 of model establishment, 40 successfully modeled mice were selected and randomly divided equally into 8 groups, which were injected with different mRNA vaccines, proteins or PBS respectively. The groups include: PBS group, Luc mRNA (10 µg) group, L+M+S mRNA (5+5+5 µg) group, L+S mRNA (5+5 µg) group, L mRNA (5 µg) group, S mRNA (5 µg) group, LMS VLP (5 µg) plus 125 µg Alhydrogel^{®} adjuvant group, and Pre-S peptide (5 µg) plus 125 µg Alhydrogel^{®} adjuvant group. Mice were immunized via intramuscular injection on Day 0 and Day 15 of immunization. Orbital blood collection was performed on Day 0 and once every subsequent week until the end of the experiment to obtain serum samples. Serum HBsAg and HBsAb were detected by chemiluminescence assay using Abbott reagents, and serum HBV DNA was quantified using the Sansure Hepatitis B Virus Nucleic Acid Quantitative Detection Kit.

The results are shown in Figure 14. Within the 21-day window following the successful establishment of the pAAV HBV1.2 mouse model, immunization with combinations of three or two mRNAs exerted a significant inhibitory effect on HBV surface antigen and envelope antigen. Unlike the HBV Tg model, immunization with L mRNA alone also exhibited inhibitory effects. This may be attributed to the relatively low levels of hepatitis B antigens in this model and the marked decline in antigen expression over a short period of time. FACS and WB results of *in vitro* mRNA transfection in Example 4 revealed that L mRNA achieved moderate expression in the early phase after transfection, which enables the activation of antibody generation to a certain extent in this model. In addition, antibody activation exhibited slight differences from antigen. At day 14 after the primary immunization, only immunization with combinations of three or two mRNAs significantly triggered robust high-level antibody generation. High-level antibodies were not generated until day 21 following single immunization with either L mRNA or S mRNA.

### Example 7 Study on the Therapeutic Effect of mRNA Vaccine in rAAV HBV1.3 Mouse Model

Recombinant AAV8 virus carrying 1.3 copies of the full-length HBV genome was intravenously injected into mice at a dose of 1×10¹⁰ or 5×10¹⁰ vg/mL. A stable HBV persistent replication model can be established 4 weeks after injection, which generally maintains high levels of serum and viral indicators for 3 months.

The construction of the rAAV HBV1.3 mouse model is described in detail as follows: each mouse was injected via the tail vein with rAAV HBV1.3 viral solution at a dose of 1×10¹⁰ or 5×10¹⁰ vg/mL. A stable HBV replication model was established 4 weeks after model construction (the model construction procedure is shown in Figure 21a).

After successful establishment of a model, 30 successfully modeled mice were selected and evenly divided into 6 groups, which were injected with different mRNA vaccines, proteins or PBS respectively. The groups included: PBS, L+M+S mRNA (5+5+5 µg), L+S mRNA (5+5 µg), L mRNA (5 µg), S mRNA (5 µg), and LMS VLP (5 µg) plus 125 µg Alhydrogel^{®} adjuvant. The specific injection timeline is shown in Figure 21a. Mice were immunized via intramuscular injection on Day 0 and Day 15 of immunization. Orbital blood collection was performed on Day 0 and once every subsequent week to obtain serum samples until the end of the experiment at 3 months. Serum HBsAg and HBsAb were detected by chemiluminescence assay using Abbott reagents. Sansure HBV nucleic acid quantitative detection kit was used to measure serum HBV DNA. Figure 20 shows the dynamic changes in HBsAg, anti-HBsAg antibody, HBeAg antigen and HBV DNA levels after immunization in the model experiment with 1×10¹⁰ vg/mL, while Figure 21 shows the variations in HBsAg, anti-HBsAg antibody and HBeAg antigen levels following immunization in the model experiment with 5×10¹⁰ vg/mL.

### Example 8 mRNA Vaccine Activates Antigen-Specific T Cell Immune Responses in HBV Tg Mice

In this Example, an experiment was performed to stimulate splenic T cells isolated from the animal model in Example 5 using LMS co-expressed mouse cells, to investigate whether the mRNA vaccine activates Pre-S native antigen-dependent T cell immune responses.

The specific procedures are as follows: (1) pCDNA3.1 (+)-: L protein, pCDNA3.1 (+): M protein and pCDNA3.1 (+): S protein expression plasmids carrying different resistance genes were constructed, the plasmids were linearized, and then the three plasmids were co-transfected into mouse embryonic fibroblast Balb/3T3 cells using Lipo3000. At 96 h post-transfection, resistance gene screening was performed to enrich antigen-positive cells. The screening was performed for two weeks followed by FACS detection (Figure 19, panel a), and the cells were prepared for incubation with T cells; (2) mice with favorable therapeutic effects obtained in Example 5 were sacrificed. The spleens were harvested and ground, lymphocytes were isolated via percoll density gradient centrifugation, and T cells were further purified using CD3 magnetic beads. The harvested T cells were counted and diluted with cell culture medium; (3) T cells from each group were added to antigen-expressing Balb/3T3 cells at a ratio of 1:5 and incubated for 12 hours. Cells of each group were harvested by rinsing with pre-cooled PBS, cells were treated with fixative solution, and washed with PBS. Cells were incubated with anti-CD3e antibody, followed by PBS washing. Cells were permeabilizd with True-Phos^{™} Perm Buffer (BioLegend) and incubated with anti-IFN γ antibody. After PBS washing, flow cytometry was adopted to detect the proportion of IFN γ-positive cells among CD3-positive cells (Figure 19b).

### Example 9 Detection of Specific Antibodies Against Pre-S Native Antigen Epitopes in HBV Tg Mice Immunized with mRNA Vaccine

In this Example, IgG in the serum of HBV Tg mice immunized with the purified mRNA vaccine was purified. Anti-S antibodies were removed via S antigen binding screening to eliminate the interference of anti-S IgG antibodies. The binding of the screened IgG to HBV viral particles was detected to determine whether immunization with the mRNA vaccine triggers the generation of specific antibodies targeting the native re-S antigen epitope in HBV Tg mice.

The specific procedures are as follows: (1) In Example 5, from the 3^{rd} immunization with mRNA vaccines to week 14, sera were collected from mice in the PBS group, Luc mRNA group, L+M+S mRNA group, L+S mRNA group, L mRNA group, S mRNA group, LMS VLP group and Pre-S group every two weeks. After the cumulative serum volume of each group reached 1 mL, IgG was purified using Protein A columns; (2) Diluted Pre-S peptides, recombinant HBsAg proteins and LMS VLP particles were separately used to coat microtiter plates overnight at 4 °C, washed with PBS, and blocked with goat serum. 1 µg of IgG sample was added to each well, incubated overnight at 4 °C, and washed with PBST. HRP anti-mouse antibody was added, and incubated at 37 °C for 2 hours, and washed with PBST. TMB chromogenic solution was added for the color reaction, followed by the addition of 2 M H₂SO₄ stop solution. The values of OD450-OD603 were measured with a microplate reader. (3) Streptavidin magnetic beads were combined with biotin-labeled recombinant HBsAg protein to bind and remove anti-S IgG; (4) Diluted serum from hepatitis B patients (containing HBV particles), Pre-S peptides and recombinant HBsAg proteins were separately used to coat microtiter plates overnight at 4 °C, washed with PBS, and blocked with diluted normal human serum. Then 1 µg of IgG sample screened by HBsAg-magnetic beads was added and incubated overnight at 4 °C, and washed with PBST. HRP anti-mouse antibody was added and incubated at 37 °C for 2 hours, and washed with PBST. TMB chromogenic solution was added for color development, followed by 2 M H₂SO₄ stop solution, and OD450-OD603 values were detected by a microplate reader.

The experimental results are shown in Figure 15. Compared with the control group, all the HBV Tg mice immunized with L+M+S mRNA, L+S mRNA and LMS VLP generated IgG antibodies against Pre-S peptide and HBsAg. S mRNA and Pre-S respectively triggered the generation of antibodies targeting HBsAg and Pre-S peptide (Figure 11c, Figure 15).

### Example 10 Analysis of Serum Antibodies Inhibiting In Vitro Cell Line Infection in Mice Immunized with Different mRNA Vaccines

In this Example, C57 mice were immunized with mRNA, and serum samples were collected (the exact concentration of effective neutralizing antibodies was unknown). An *in vitro* model of HBV infecting the HepG2-hNTCP cell line was adopted to detect and compare the *in vitro* inhibitory effects of immune sera at different concentration gradients against HBV infection in the HepG2-hNTCP cell line. By measuring HBeAg levels, the HBeAg inhibition rates of different serum samples at varying concentrations were determined, and the corresponding dilution folds and working concentrations of serum samples when the inhibition rate against HBV infection reached 50% (IC50) were calculated. The experimental procedures are as follows: (1) HepG2-hNTCP cell line were prepared; (2) the HepG2-hNTCP cell line was infected with replication-competent and infectious HBV under conditions with the addition of HBIg positive control antibody (Figure 22a), serum samples with different concentration gradients (Figure 22c, Figure 22d, Figure 22e), or without serum samples (Figure 22b), with three replicates for each group. Groups 1-4 serum groups were sera from mice immunized with PBS, LMS mRNA, LS mRNA and S mRNA, respectively; (3) Culture supernatants were collected on Day 7 after HBV infection; (4) HBeAg levels in the supernatants were detected. Meanwhile, the calculated results were compared and converted with the IC50 concentration value of the HBIg positive control antibody to obtain the specific concentration of effective neutralizing antibodies in undiluted serum samples (Figure 22f). The experimental results were used to evaluate the neutralizing capacity and effective antibody concentration of serum samples.

### Example 11 Toxicological Analysis of Mice Immunized with Different Doses of mRNA Vaccine

Male ICR mice aged 5 to 6 weeks were selected and injected intramuscularly on Day 0 and Day 3, respectively. The injection doses were 1.5 µg, 7.5 µg, or 37.5 µg of LMS mRNA (mass ratio = 1:1:1), and an equal volume of PBS was used as the control (n=8). During the experiment, the mortality, appearance, and behavioral activity of mice were observed every 3-4 days, and body weight changes were recorded (Figure 23, panel a) until 21 days after the second administration.

At the end of the experiment, mouse serum was isolated, and the weights of major organs (including the heart, liver, spleen, lung and kidney) were measured (Figure 23, panel b). Meanwhile, liver function indicators (Figure 24, panel a) and renal function indicators (Figure 24, panel b) in mouse serum were detected. Liver function indicators included alanine transaminase (ALT) and aspartate transaminase (AST); and renal function indicators included urea (UREA), uric acid (UA) and creatinine (CR). After the organs were rinsed with PBS, they were fixed in 4% paraformaldehyde for 2 days, followed by H&E staining for pathological analysis (Figure 25).

All documents mentioned in the present invention are incorporated herein by reference to the same extent as if each individual document was specifically and individually indicated to be incorporated by reference. In addition, it should be understood that those skilled in the art can make various changes and modifications to the present invention after reading the above teaching contents of the present invention, and such equivalent forms shall also fall within the scope defined by the appended claims of the present application.

## Claims

1. A vaccine composition, comprising:
a first polynucleotide N_{L} encoding hepatitis B virus surface antigen L protein; and
a second polynucleotide N_{S} encoding hepatitis B virus surface antigen S protein.

2. The vaccine composition according to claim 1, wherein the molar ratio of N_{L} to N_{S} is 1:10 to 10:1, preferably 1:5 to 5:1, more preferably 1:2 to 2:1.

3. The vaccine composition according to claim 1, wherein the vaccine composition further comprises a third polynucleotide N_{M} encoding hepatitis B virus surface antigen M protein.

4. The vaccine composition according to claim 1, wherein the transcription start site of M protein and S protein in the L protein coding sequence is mutated, so that only the L protein can be translated.

5. The vaccine composition according to claim 1 or 3, wherein the polynucleotide is mRNA.

6. The vaccine composition according to claim 5, wherein the mRNA sequence encoding the L protein in N_{L} is shown in SEQ ID NO: 4, the mRNA sequence encoding the M protein in N_{M} is shown in SEQ ID NO: 5, and the mRNA sequence encoding the S protein in N_{S} is shown in SEQ ID NO: 6.

7. The vaccine composition according to claim 1, wherein the vaccine composition is a lipid nanoparticle, which comprises an outer liposome layer and the polynucleotide encapsulated therein.

8. An engineered host cell, wherein the engineered host cell expresses membrane-bound hepatitis B virus surface antigen L protein and S protein; or expresses membrane-bound hepatitis B virus surface antigen L protein, S protein and M protein.

9. The engineered host cell according to claim 8, wherein the host cell is obtained by transfection with the vaccine composition according to claim 1 or 3.

10. A method for preparing the engineered host cell according to claim 8, comprising a step of:
transducing the vaccine composition according to claim 1 into an immune cell, so as to obtain the engineered host cell.

11. Use of the vaccine composition according to claim 1 or the engineered host cell according to claim 8 in the preparation of a medicament, wherein the medicament is used in a subject for:
(a) preventing hepatitis B virus infection;
(b) inducing the production of antibodies specific for hepatitis B virus; and/or
(c) treating chronic hepatitis B infection.

12. The use according to claim 11, wherein the chronic hepatitis B is immune-tolerant chronic hepatitis B.

13. A medicament for treating chronic hepatitis B infection, wherein the medicament comprises the vaccine composition according to claim 1 or the host cell according to claim 8.

14. A method for preventing and/or treating hepatitis B, comprising a step of:
administering the vaccine composition according to claim 1 or the engineered host cell according to claim 8 to a subject in need thereof.

15. A method for generating anti-hepatitis B virus antibodies, comprising a step of:
immunizing an animal with the vaccine composition according to claim 1 or the engineered host cell according to claim 8, thereby generating the anti-hepatitis B virus antibodies.
